# EUROPEAN PATENT APPLICATION

(11) **EP 1 632 245 A1**
(43) Date of publication of application: **08.03.2006**
(21) Application number: 04020912.4
(22) Date of filing: 02.09.2004
(51) Int. Cl.: A61K 38/17, A61P 3/10

(54) **ICA512 couples insulin secretion and gene expression in Beta-cells**

(71) Applicant: Technische Universität Dresden Medizinische Fakultät Carl Gustav Carus, 01307 Dresden (DE)
(72) Inventor: Trajkovski, Mirko, 01099 Dresden (DE); Mziaut, Hassan, 01279 Dresden (DE); Solimena, Michele, 01326 Dresden (DE)
(74) Representative: Vossius & Partner

(57) **Abstract**

The present invention relates to a method for stimulating expression of peptide hormones in peptide-hormone secreting endocrine cells or neurons comprising the step of promoting in said cells or neurons the presence or activity (aa) of (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally of µ-calpain or a fragment or derivative thereof having µ-calpain function; or (b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons. Additionally, the present invention relates to a method of promoting cell proliferation of peptide-hormone secreting endocrine cells or neurons comprising the step of promoting in said cells or neurons the presence or activity (aa) of (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally of µ-calpain or a fragment or derivative thereof having µ-calpain function; or (b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons. It is preferred in accordance with the invention that said endocrine cells are β-cells and that said peptide hormone is insulin.

## Description

The present invention relates to a method for stimulating expression of peptide hormones in peptide-hormone secreting endocrine cells or neurons comprising the step of promoting in said cells or neurons the presence or activity (aa) of (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally of µ-calpain or a fragment or derivative thereof having µ-calpain function; or (b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons. Additionally, the present invention relates to a method of promoting cell proliferation of peptide-hormone secreting endocrine cells or neurons comprising the step of promoting in said cells or neurons the presence or activity (aa) of (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally of µ-calpain or a fragment or derivative thereof having µ-calpain function; or (b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons. It is preferred in accordance with the invention that said endocrine cells are β-cells and that said peptide hormone is insulin. Further embodiments of the invention are recited in the appended claims.

In the specification, a number of documents is cited. The disclosure content of these documents, including manufacturers' manuals is herewith incorporated by reference in its entirety.

Neuroendocrine cells, including the insulin-producing β-cells of the endocrine pancreas, modulate physiological activities through the regulated secretion of peptide hormones and neuropeptides. These signaling molecules are stored intracellularly within organelles termed secretory granules (SGs) that fuse with the plasma membrane in response to different stimuli. This process, commonly referred to as regulated exocytosis, is responsible for the release of peptide hormones and neuropeptides in the extracellular space and, in the case of peptide hormones, into the systemic circulation. An open question is whether feedback-signaling mechanisms exist which allow neuroendocrine cells to constantly monitor their regulated secretory activity and adjust accordingly their gene expression profile and hormone biosynthesis.

A large variety of peptide hormones and neuropeptides is known in the art. These include, inter alia, amylin and insulin. Insulin is the peptide hormone responsible for maintaining glucose homeostasis and a deficit of its production, secretion or signaling can cause diabetes. Due to the increasing numbers of diabetes patients particularly in the Western world over the last decades, regulation of insulin expression has become a focus of medical research.

ICA512, also known as IA-2, is a major target of autoimmunity in type 1 diabetes, associated with insulin SGs, and neurosecretory granules in general (I) (1). This type I transmembrane protein, (2, 3), belongs to the receptor protein tyrosine phosphatase (PTP) family due to the presence of a single PTP domain, which however lacks phosphatase activity (4). Deletion of ICA512 was shown to impair insulin secretion in mice (5), but the reason for this phenotype is unclear.

ICA512 is released from a precursor, pro-ICA512. Pro-ICA512 is a glycoprotein of 110 kD (6) synthesized in a glucose-regulated fashion (7, 8). Processing of its ectodomain along the secretory pathway by a furin-like protease converts pro-ICA512 into its mature transmembrane form of 65 kD (ICA512-TMF), which is enriched on SGs (1). Stimulation of SG exocytosis prompts the translocation of ICA512 to the plasma membrane (1) and reduces the levels of ICA512-TMF because of the Ca²⁺-dependent cleavage of its cytoplasmic domain, presumably by µ-calpain (7).

Among the 14 mammalian cysteine proteases of the calpain family, only µ-calpain is activated by micromolar Ca²⁺ concentrations (11), i.e. within the range levels elicited by physiological stimuli near the plasma membrane in order to trigger SG exocytosis. Accordingly, stimulation of secretion promoted calpain activity (7). Furthermore, µ-calpain cleaved in-vitro recombinant human ICA512 cytoplasmic domain between residues 658-659, few residues downstream of a PEST sequence (7). PEST regions may serve as proteolytic signals and are often present within calpain substrates (12).

Some valuable information regarding the regulation of insulin that may well have an impact on the development of cures relating to diseases interrelated with the dereguation of expression or secretion of neuropeptides has recently been compiled. In particular, it has been found that post transcriptional mechanisms are responsible for rapidly up regulating the expression of insulin and SG proteins in response to glucose. Specifically, it was shown that stimulation of β-cells promotes the nucleocytoplasmic translocation of polypryimidine tract binding protein (PTB), which in turn binds mRNAs encoding SG proteins, including insulin and ICA512, and increases their stability and translation (8).

In conclusion, a number of reports have elucidated the regulation of expression and secretion of peptides contained in secretory granules. Some of these reports are expected to guide the development of new approaches in the prevention or treatment of diseases interrelated with the deregulation of expression or secretion of such peptides. Yet, due to the importance of finding new and promising medicaments or lead compounds for medicaments for the mentioned diseases and in particular for diabetes, there is a continuing need for finding such medicaments or lead compounds. The solution to this need, or technical problem, is achieved by providing the embodiments characterized in the claims.

Accordingly, the present invention relates to a method for stimulating expression of peptide hormones in peptide-hormone secreting endocrine cells or neurons comprising the step of promoting in said cells or neurons the presence or activity (aa) of (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally of µ-calpain or a fragment or derivative thereof having µ-calpain function; or (b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

The method of the invention may be carried out in vivo, in vitro or ex vivo. This holds also true for other embodiments of the method of the invention addressed further below.

The options (a) comprising (aa) and (ab) or (b) as recited above in accordance with the main embodiment of the invention, as well as with respect to further embodiments of the invention discussed further below are not intended to be mutually exclusive. Rather, the options can occur simultaneously or in conjunction. Thus, in accordance with the present invention wherever several options are recited, these may be put into effect either alone or in conjunction unless stated otherwise. The same holds true for the features of the C-terminal fragment of the ICA512 protein or derivative thereof etc., i.e. the capability of the C-terminal fragment or derivative of being targeted to the nucleus, of interacting with a PIAS protein and of interacting/regulating transcriptions factors and preferably STATs. The C-terminal fragment or derivative thereof may have one, two or all three of these characteristics wherein it is preferred that all three characteristics are present. It is noteworthy that the invention also comprises embodiments wherein the C-terminal fragment or derivative thereof optionally interacts with/regulates instead of or in addition to STATs other transcription factors such as PDX-1 and RIPE3b1-Act/C1. In general, the activity of such transcription factors may be enhanced by promoting their nuclear translocation, by preventing their export from the nucleus, by increasing their half-life and, foremost, by increasing their ability to bind DNA and co-activators of transcription.

The term "expression" is well known in the art and relates to the transcription and/or translation of a gene, resulting in the generation of a messenger RNA and, optionally, subsequently, its translation into a protein. In accordance with the present invention, it is preferred that in particular the transcription of the gene is stimulated.

Expression is "stimulated", if the level of expression of the gene in question is enhanced by at least 20%, preferably at least 50%, more preferred at least 75% such as at least 100% of a level that is reached without the promotion of the presence or activity as recited above. Most preferred, the level of expression is enhanced by at least 200% such as at least 500% without intending to mean that these values constitute the upper limits of enhancement. As a reference level, non-stimulated cells may serve such as pancreatic β-cells that display only a basal level of insulin production.

The term "peptide hormone-secreting endocrine cells or neurons" refers to any mammalian and preferably human cell that naturally produces and secretes peptide hormones or neuropeptides (neuropeptides being included in accordance with this invention within the group of peptide hormones). Also included are precursors of such cells as well as cells which in their normal context in the body do not secrete such peptides but which have been manipulated to secrete such peptides, for example by genetic engineering. In principle, one option in this regard envisages reprogramming a somatic cell to transdifferentiate into a peptide-secreting endocrine cell or a neuron. It is preferred in accordance with the present invention that peptide hormone-secreting endocrine cells or neurons are human cells that naturally produce and secrete peptide hormones or neuropeptides.

The term "promoting the presence" in general means that a certain level (aa) of (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally of µ-calpain or a fragment or derivative thereof having µ-calpain function; or (b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons is maintained in the cytoplasm of the recited cells or neurons. Accordingly, the invention envisages that, in one alternative, an existing level of any of the above recited compounds in the cytoplasm is maintained or essentially maintained, such as maintained to at least 70%, preferably to at least 80%, more preferred to at least 90% and most preferred to at least 95% such as 100%. Important options to maintain an existing level of said compounds in the cytoplasm are described in connection with preferred embodiments of the method of the invention further below. In a second alternative, ICA512 or a derivative thereof having ICA512 function or a fragment thereof that may be cleaved by µ-calpain giving rise to a C-terminal fragment of ICA512 or any derivative or fragment thereof as recited above is actively produced inside the cell and/or shifted from the cytoplasm into the nucleus. In this alternative, several options are possible how the level of ICA512 or any derivative or fragment thereof as recited above, in addition or in the alternative, may be enhanced in a cell, in particular in the cytoplasm or nucleus. For example, if the cell naturally does not produce any of said compounds, production may be stimulated by introducing a vector capable of expressing at least one of said compounds into said cell. Important options how the level of said compounds may be enhanced are discussed in connection with preferred embodiment further below. Further to the above, it is to be understood that the term "enhancing" in connection with the production of ICA512 or any derivative or fragment thereof as recited above relates both to the enhancement of levels of any of said compounds already existing in the cytoplasm or in the nucleus and to the de novo production thereof in a cell. The level of said compounds required in a cell that is necessary for stimulating expression may vary from cell type to cell type. In any case and as regards the cell or neuron, it is preferred that said cell or neuron is in a stimulated stage, i.e. secretes said peptide-hormones. In the case of pancreatic β-cells, stimulation may be effected by contact with or addition of glucose.

As regards the optional promotion of the presence or activity of µ-calpain or the recited fragments or derivatives thereof, the following should be noted: Generally, it is advantageous or even necessary to promote the presence or activity of µ-calpain etc. (wherein "etc." means the respective above recited fragments or derivatives) if µ-calpain is per se not expressed in the respective cells or neurons or expressed only to such a low extent that a turnover of the ICA512 protein etc. present in said cells or neurons is not guaranteed, in particular, if the level of expression of the substrate is even further enhanced in accordance with the method of the invention. The level of µ-calpain in a cell may be measured by biochemical means, such as staining with labelled anti-µ-calpain antibodies or by measuring the turnover of a µ-calpain substrate, preferably of ICA512 in extracts of the respective cells according to classical biochemical techniques or by using the antibodies recited herein that differentiate between full length ICA512 and the fragments generated by µ-calpain. On the other hand, the promotion of said presence or activity may not be required, if amounts sufficient to cleave the ICA512 or fragments or derivatives thereof recited above, upon promotion of the presence or activity of said ICA512 etc. are present in the cell, even if a de novo synthesis of µ-calpain or a catalytically active fragment or derivative thereof does not occur in the cell and the overall level of the protein etc. is thus not maintained but decreases. Furthermore, if a peptidomimetic is employed, irrespective of its length (e.g. even when it corresponds to full-length ICA512), µ-calpain may not be necessary in said cell.

The term "promoting the [...] activity" refers, as was explained in conjunction with the term "promotion of the presence" to the essential maintenance or an increase of the activity. An increase of the activity may be effected, for example, by site-directed mutagenesis and the subsequent testing for an increased activity.

"ICA512" refers to, in accordance with this specification, the mature transmembrane protein "Islet Cell Autoantigen 512" also referred to as "IA-2" or "protein tyrosine phosphatase N (PTPN)" having a level of identity of at least 30%, preferably at least about 35%, more preferred at least 50% and most preferred and least 75% such as 85% with the amino acid sequence displayed in SEQ ID NO.: 1 which reflects the human ICA512 sequence. This sequence is also available as entry # Q16849 from the NCBI protein database. This amino acid sequence, in accordance with the invention, is preferably encoded by the nucleotide sequence of SEQ ID NO.: 2 which is available as entry # L18983 from the NCBI nucleotide database. It is to be understood that the various ICA512 proteins having different degrees of identity and subsumed under the term "ICA512" solely denote naturally occurring proteins from other species that may, in the scientific literature, bear a name different from ICA512. Thus, a mouse ICA512 protein, (NCBI entry #I48721) has been identified that bears 86.7% identity with the human sequence at the amino acid level. Similarly, rat (NCBI entry #Q63259, 86.82% identity), Danio rerio (NCBI partial sequence # AF190144.1, 75.97% identity), and C. elegans (NCBI entry # CAB52188, 38.10% identity) proteins have been identified. All these proteins have the following functions in common: 1) they all include a single atypical protein tyrosine phosphatase (PTP) domain in their cytosolic tail that lacks phosphatase activity towards conventional tyrosine phosphatase substrates, 2) they are most abundant in neuroendocrine cells; 3) they are enriched on SGs . They may all be used in accordance with the method of the present invention.

The term "derivative of ICA512 having ICA512 function" relates to a protein (the term "protein" in connection with this specification having the same meaning as the term "polypeptide") that is derived from naturally occurring ICA512, e.g., by post-translational modifications that are effected by eukaryotic cells other than the cells the protein naturally occurs in or by lack of post-translational modifications (in case the protein is produced in prokaryotic organisms), by chemical modification, by peptidomimetics etc. to name a few important options. In all these options, the ICA512 function is retained. "ICA512 function" means, in accordance with this invention, to include a PTP like-domain, the capability to be cleaved by µ-calpain and to sequester a C-terminal portion that retains the capacity to be targeted to the nucleus, and/or bind proteins of the PIAS (Protein Inhibitor of Activated STAT) family, and/or instigate or enhance the expression of genes encoding peptide-hormones by modulating the activity of transcription factors, such as Signal Transducer and Activator of Transcription (STATs) as discussed herein above. Included within the term "derivative" are also/thus molecules that have the above identified biological function and that are (semi)synthetically or recombinantly produced without assuming first the naturally occurring structure of ICA512 which is then subsequently modified, e.g. by post-translational modifications that are effected by eukaryotic cells other than the cells the protein naturally occurs in. This is the case if the derivative is a peptidomimetic. Alternatively, the term includes fragments of the full-length protein that are modified as outlined above and that retain the function specified above. Modifications referred to above (in particular post-translational and chemical) include: acetylation, acylation, amidation, ADP-ribosylation, glycosylation, GPI anchor formation, covalent attachment of a lipid or lipid derivative, methylation, myristylation, pegylation, prenylation, phosphorylation, ubiqutination and sumoylation. Derivatives of the above protein include further fusion proteins wherein the two fusion partner may, e.g., be fused via a (flexible) linker such as a glycine linker or a gly-ser linker or wherein the protein is fused to a tag such as His-tag, FLAG-tag, myc-tag etc.

The term "pro-form" of ICA512 or "pro-ICA512" refers to a glycoprotein of 110 kD (6) synthesized in a glucose-regulated fashion (7, 8). Processing of its ectodomain along the secretory pathway by a furin-like protease converts pro-ICA512 into its mature transmembrane form of 65 kD (ICA512-TMF), which is enriched on SGs. (1). The pro-form may be derived from natural sources or be (semi)synthetically produced. Preferably, it is recombinantly produced. Recombinant production may be effected in any suitable host cell such as eukaryotic host cells such as a mammalian host cell or a yeast cell or an insect cell. Alternatively, a bacterial host cell may be used. For such purpose, the gene encoding the protein in question is normally cloned into a suitable vector, the host cell transfected with said vector, and the protein produced in the host cell. After production and depending on the expression system employed, the protein may be recovered from the host cell by breaking up the same or from the culture supernatant (see, for example, Sambrook et al, "Molecular Cloning, A laboratory manual", 2^{nd} edition, CSH Press 1989, Cold Spring Harbor). The pro-form may have any modification that was outlined in conjunction as long as it retains its ability to be cleaved by µ-calpain, which gives rise to a functional C-terminus that targets the nucleus and enhances expression of a peptide-hormone. Tests how this function can be tested can be performed in accordance with the teachings of this specification.

"µ-calpain " is a mammalian cysteine protease of the calpain family that occurs in the cytoplasm. It is the only member of this family known to date which is activated by micromolar concentrations of Ca++ ions, i.e within the range of Ca++ concentrations which can be physiologically reached in the cytosolic compartment. It can also be activated by phospholipids. Proteins with PEST sequences constitute preferential targets for µ-calpain (Rechsteiner, M. Semin. Cell Biol. 1 (1990), 433-440; Mykles, D.M., Int. Rev. Cytol. 194 (1998), 157-289). It is important in accordance with the invention that the enzyme or fragment ot derivative thereof is catalytically active and can thus cleave ICA512.

A "derivative of µ-calpain having µ-calpain function" may contain any modification as outlined herein above for derivatives of ICA512 as long as the function of µ-calpain is retained. This function necessarily includes the capacity to cleave mature transmembraneous ICA512 (or an appropriate derivative of fragment thereof) between positions 658 and 659 of the mature full-length protein. "Fragments of µ-calpain" also necessarily retain this function. Retention of the required function may be assessed by employing tests as described in this specification or in (7).

The term "a C-terminal fragment of ICA512 [...] or a derivative thereof which has the capability of being targeted to the nucleus" refers to any C-terminal portion of this protein that is derived or sequestered from ICA512 which is optionally modified as outlined above and that has the capacity to move from the cytoplasm into the nucleus. The C-terminal fragment may be obtained from naturally occurring full-length ICA512 by cleavage with a protease such as µ-calpain. In one alternative (not excluding further alternatives to which the skilled artisan may revert) the fragment is synthesized chemically or produced recombinantly. The capacity to be targeted to the nucleus can be determined by the skilled artisan on the basis of the teachings of this specification without further ado. In accordance with this invention, the most N-terminally located amino acid residue of the C-terminal fragment is residue 601 of the full length sequence. Preferably, the most N-terminally located sequence is the amino acid in position 643 and most preferred, it is in position 659. Of course, any other position between amino acid residues 601 and 659 may also serve as the N-terminus. In addition, positions downstream of amino acid 659 may serve as the N-terminus. With the teachings provided in the specification, the skilled artisan is in the position to determine without undue burden whether an N-terminal amino acid position downstream of position 659 and which position gives rise to a C-terminal fragment having the function of being targeted to the nucleus and stimulating insulin expression. Accordingly, all these embodiments are encompassed by the scope of the present invention.

A "C-terminal fragment of ICA512 or a derivative thereof which has the capability of interacting with a PIAS protein" may be the same fragment as before and may thus have the additional function of interacting with a PIAS protein. The term "PIAS protein" refers to a family of nuclear proteins that act as a E3-ligases in the sumoylation reaction, which consists in the post-translational modification of proteins by the covalent attachment of a SUMO peptide to a cytosolic lysine in the primary amino acid sequence of target proteins. PIAS proteins can also modulate the activity of many transcription factors, including STATs. Alternatively, the fragment may be distinct in function from the foregoing C-terminal fragment in that the foregoing fragment is targeted to the nucleus but does not (necessarily) bind to PIAS proteins. In any case, the fragment may be modified as above with the proviso that it necessarily binds to PIAS proteins. Binding may be measured using the experimental set-up described in this specification. "STATs", such as STAT-5, are proteins that serve the dual function of signal transducers and activators of transcription in cells exposed to signalling polypeptides, such as cytokines, interleukins, growth hormone and prolactin. The STAT protein family includes STAT-3 and STAT-5 which are preferably employed in accordance with the present invention. Tyrosine phosphorylation of cytosolic STATs promotes their dimerization and nuclear translocation, thereby allowing these proteins to bind regulatory DNA elements and promoting gene transcription. STAT-5, in particular, stimulates insulin gene expression and β-cell proliferation upon stimulation with growth hormone and prolactin (Nielsen et al., 2001). STAT-5 activity is activated by tyrosine phosphorylation (Herrington et al., 2000), while it is inhibited by PIAS3 (Rycyzyn and Clevenger, 2002).

A "fragment of ICA512 that may be cleaved by µ-calpain giving rise to a C-terminal fragment of ICA512 or a derivative thereof [which] has the capability of being targeted to the nucleus" is thus a fragment that extends N-terminally over the C-terminal fragment having been defined above. In fact, such a fragment could extend up to amino acid position 2 of ICA512 - N-terminally. Optionally, the fragment gives rise to a C-terminal fragment that alternatively or additionally has the capability of interacting with a PIAS protein or of interacting with/regulating transcription factors such as STATs in said cells. As stated above, the interaction with PIAS proteins results in the sumoylation of the C-terminal fragment or derivative which in turn is expected to lead to enhance the nuclear translocation/retention of the transcription factors and/or their stability. Whereas the applicant does not wish be bound by any theory, binding of the C-terminal fragment etc. to PIAS is expected to modulate its ability to bind transcription factors and/or its sumoylating activity.

Similarly, "derivatives" of these fragments as recited above may give rise to derivatives of said C-terminal fragments of ICA512 that have at least one, preferably two and more preferred all three of the above recited characteristics.

The term _{"}a fragment or derivative [...] [which] has the capability of enhancing the nuclear levels or tyrosine phosphorylation or DNA binding activity of STATs in said cells or neurons" refers to fragments or derivatives according to any of the foregoing embodiments which exhibit at least one of these three biological functions on STAT proteins in the recited cells. Preferably, the fragment or derivative displays all three different activities on STATs. It is further preferred, that the fragment or derivative additionally has the function of interacting with a PIAS protein. Further preferred is that the fragment or the derivative also has the functions or features of the other fragments or derivatives recited in the main embodiment of the invention.

In accordance with the present invention it was surprisingly found that C-terminal fragments of ICA512 are targeted to the nucleus and, within the nucleus stimulate expression of peptide hormones in peptide-hormone secreting endocrine cells or neurons. In addition, it was found that said C-terminal fragment which was experimentally generated by cleavage with µ-calpain interacts with PIAS proteins and regulates the activity of STAT proteins. Whereas the Applicant does not wish to be bound by any scientific theory, it is well possible that binding of said C-terminal fragment to said PIAS proteins has a direct impact on the stimulation of expression of said peptide hormones, presumably but not necessarily through the regulation of the transcription factors of the STAT family.

On the basis of these surprising findings, cells including neurons can now be stimulated for expression of the desired peptide hormone. In this manner, a reduced level of such peptide-hormones leading to a disease or disorder or being suspected of leading to a disease or disorder can now be enhanced. The enhancement is believed to counterbalance the effects of the disease or to prevent or cure the disease.

In essence, the method of the invention envisages various setups. For example, the mature ICA512 (or a derivative or fragment thereof as defined herein above) may be expressed at high levels in the cell and subsequently cleaved by µ-calpain (or a fragment or derivative thereof as defined herein above). In this manner, increased amounts of C-terminal fragments of ICA512 may be retargeted to the nucleus and stimulate or enhance expression of peptide hormones as desired. Alternatively or additionally (e.g., if high levels of ICA512 etc. but unsufficiently low levels (see above) or no µ-calpain is/are expressed in the cell), µ-calpain function may be enhanced, for example, by constitutive expression of µ-calpain. This can be done, for example, in cells that are transfected with a vector comprising the gene for µ-calpain under the control of a promoter that is constitutively active or may be specifically induced in suitable cells. The function of µ-calpain to cleave ICA512 may be enhanced, in a further alternative, by increasing the specific cleavage activity, for example by site directed mutagenesis or by inhibiting the endogenous calpain inhibitor calpastatin. Proteases with an enhanced activity level may be selected in cleavage assays as described in this specification. Alternatively, the invention envisages methods of promoting the activity of ICA512, for example by selecting ICA512 mutants that are more stable, that bind better PIAS, that increase STAT activity to a higher level and in any event, that are more effective in enhancing peptide-hormone gene expression. Naturally, any combination of the above options as well as the inclusion of further options that are not specifically mentioned here is within the scope of the invention.

As regards option (b), the method of the invention does not rely on the cleavage of ICA512 by µ-calpain. Rather, the C-terminal fragment of ICA512 which has the capability of being targeted to the nucleus and/or which has the function/capability of interacting with a PIAS protein and/or that has the function/capability of interacting with/regulating transcription factors such as STATs can be provided within the cells. Enhancing the abundance of the C-terminal fragment is believed to be suitable to stimulate expression of the peptide hormones in the cells mentioned. Activity of the fragment may be increased, for example, by enhancing the binding affinity to PIAS proteins/STATs.

Some of the options recited above how the method of the invention may be put into practice will require the formulation of at least one of the compounds mentioned into a pharmaceutical composition. This holds equally true for other embodiments of the invention referred to further below. The pharmaceutical composition may, in general, be in solid, liquid or gaseous form and may be, inter alia, in a form of (a) powder(s), (a) tablet(s), (a) solution(s) or (an) aerosol(s).

The pharmaceutical composition may be particularly useful for the treatment of any disease that is causally interrelated with lack of production/expression, diminished production/expression or retarded production/expression of neuropeptides contained in secretory granules.

The pharmaceutical composition may further comprise pharmaceutically acceptable carriers, excipients and/or diluents. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the pancreas or into a brain artery or directly into brain tissue. The compositions may also be administered directly to the target site, e.g., by biolistic delivery to an external or internal target site, like the pancreas or brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 1 µg to 10 mg units per kilogram of body weight per minute.

Progress can be monitored by periodic assessment. The compositions of the invention may be administered locally or systemically. Preparations for parenteral administration include sterile aqueous or non aqueous solutions, suspensions, and emulsions. Examples of non aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti oxidants, chelating agents, and inert gases and the like. Furthermore, the pharmaceutical composition may comprise further agents depending on the intended use of the pharmaceutical composition.

Additionally, a nucleic acid molecule and preferably a DNA molecule encoding said compound(s) (wherein the term "compound" also refers to the mentioned fragments or derivatives) may formulated into a pharmaceutical composition. The nucleic acid molecule is eventually to be introduced into the desired cells. Appropriate formulations include those wherein 10⁶ to 10¹² copies of the DNA molecule, advantageously comprised in an appropriate vector are administered per dose. The vector may be, for example, a phage, plasmid, viral or retroviral vector. Retroviral vectors may be replication competent or replication defective. In the latter case, viral propagation generally will occur only in complementing host/cells.

The nucleic acid molecules (or polynucleotides) may be joined to a vector containing selectable markers for propagation in a host (see also elsewhere in this specification). Generally, a plasmid vector is introduced in a precipitate such as a calcium phosphate precipitate or rubidium chloride precipitate, or in a complex with a charged lipid or in carbon-based clusters, such as fullerens. Should the vector be a virus, it may be packaged in vitro using an appropriate packaging cell line prior to application to host cells; see, for general information in this regard, Ausubel et al., Current Protocols in Molecular Biology, Green Publishing Associates and Wiley Interscience, N.Y. (2001).

The present invention further relates to a method of promoting cell proliferation of peptide-hormone secreting endocrine cells or neurons comprising the step of promoting in said cells or neurons the presence or activity (aa) of (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally of µ-calpain or a fragment or derivative thereof having µ-calpain function; or (b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

The term "promoting cell proliferation of peptide-hormone secreting endocrine cells or neurons" refers to the fact that the mentioned cells start proliferation or that proliferation is enhanced over a given and preferably a basal level wherein the basal level is advantageously that of fully differentiated cells in a individual not suffering from a disease associated with the deregulation of expression or secretion of neuropeptides from peptide-hormone secreting endocrine cells or neurons and in particular in an individual not suffering from type-1 or type-2 diabetes. In vitro, such levels may be determined using suitable cell lines. Thus, proliferation can me measured by incorporation in to DNA of radiolabeled or modified nucleic acids, such as [H3]-thymidine or bromodeoxyuridine (BrdU) into DNA. Incorporated radioactivity can be measured by β-counting, while the percentage of cells positive for BrdU can be quantified by fluorescence microscopy following immunocytochemical staining with anti-BrdU antibodies. The skilled artisan may relate the results of such assays without further ado to in vivo situations. The percentage of the increase in proliferation that is achieved in accordance with the method of the invention is advantageously at least 20%, more preferred at least 50%, even more preferred at least 75% such as at least 100% over the above mentioned level. Most preferred, the level of increase is at least 200% such as at least 500% or even at least 1000%.

This embodiment of the present invention advantageously allows for the regeneration of SGs carrying cells. As is known in the art, the transcription factor STAT-5 is regulated through proteins of the PIAS family and regulates cell growth and proliferation. The method of the invention now provides for the possibility that by triggering the promotion of any of the above mentioned compounds or combination of compounds in a cell such as in the somatic cells of interest of an individual, for example, a non-human experimental animal or a human, the population of such somatic cells of interest such as pancreatic β-cells can be increased. The medical implications of such an increase, in particular of pancreatic β-cells, are self-evident. Namely, the increase of these cells in a human that is affected by type-1 or type-2 diabetes will lead to the eradication or at least the amelioration of the disease. In non-human experimental animals such as rodents (mice, hamsters, rats etc.) the method of the invention will provide further insight into the regulation of proliferation and potentially differentiation of such cells. In addition, compounds that act synergistically with the compounds or combination of compounds to be employed in the method of the invention can be isolated, by screening methods generally available in the art that are to be adapted for the purposes of the present invention and therefore need to take into account the teachings of the present invention.

The PIAS protein to be selected may depend on the specific desire of the investigator/clinician. In a preferred embodiment of the present invention, said PIAS protein is PIASy, PIAS1, PIAS3, PIASxα or PIASxβ. When cures for the treatment of type-1 and -2 diabetes are to be developed, then the selection of PIASy is particularly preferred.

In another preferred embodiment of the present invention, the promotion comprises the transient or stable expression from an exogenously introduced vector of (aa) (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally µ-calpain or a fragment or derivative thereof having µ-calpain function; or (b) of said C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

This embodiment of the present invention may be put into practice according to conventional techniques of molecular biology including medical applications thereof such as gene therapy, and requires, optionally after cloning the gene(s) encoding the above compounds or combination of compounds (protein(s)) and insertion of the gene or the coding sequence thereof into a vector, preferably an expression vector, the introduction into a cell. The introduction is accompanied by the expression of said compound(s) from an expression vector. Examples of expression vectors useful in the method of the invention are described herein below.

Advantageously and as stated above, the polynucleotide/coding sequence is introduced into a vector and thus operatively linked to expression control sequences allowing expression in prokaryotic or eukaryotic cells or isolated fractions thereof.
Expression of said polynucleotide comprises transcription of the polynucleotide, preferably into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers. Possible regulatory elements permitting expression in prokaryotic host cells (a less preferred embodiment) comprise, e.g., the *lac, trp* or *tac* promoter in *E*. *coli,* and examples for regulatory elements permitting expression in eukaryotic host cells (the more preferred embodiment) are the *AOX1* or *GAL1* promoter in yeast or the CMV-, SV40-, RSV-promoter (Rous sarcoma virus), CMV-enhancer, SV40-enhancer or a globin intron in mammalian and other animal cells. Preferred promoters are the natural promoter of the above compounds as well as promoters allowing the tissue specific expression of those compounds such as promoters active in pancreatic β-cells or neuronal cells. Besides elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pCDM8, pRc/CMV, pcDNA1, pcDNA3 (Invitrogen), pSPORT1 (GIBCO BRL). Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (2001) N.Y. and Ausubel, Current Protocols in Molecular Biology, Green Publishing Associates and Wiley Interscience, N.Y. (2001). Alternatively, the polynucleotides or vectors can be reconstituted into liposomes for delivery to target cells.
The term "isolated fractions thereof' refers to fractions of eukaryotic or prokaryotic cells or tissues which are capable of transcribing or transcribing and translating RNA from the vector. Said fractions comprise proteins which are required for transcription of RNA or transcription of RNA and translation of said RNA into a polypeptide. Said isolated fractions may be, e.g., nuclear and cytoplasmic fractions of eukaryotic cells such as of reticulocytes. Kits for transcribing and translating RNA which encompass the said isolated fractions of cells or tissues are commercially available, e.g., as TNT reticulolysate (Promega).

Furthermore, the vector may be a gene transfer or gene targeting vector. Gene therapy, which is based on introducing therapeutic genes into cells by ex-vivo or in-vivo techniques is one of the most important applications of gene transfer. Suitable vectors, methods or gene-delivering systems for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813, Isner, Lancet 348 (1996), 370-374; Muhlhauser, Circ. Res. 77 (1995), 1077-1086; Onodua, Blood 91 (1998), 30-36; Verzeletti, Hum. Gene Ther. 9 (1998), 2243-2251; Verma, Nature 389 (1997), 239-242; Anderson, Nature 392 (Supp. 1998), 25-30; Wang, Gene Therapy 4 (1997), 393-400; Wang, Nature Medicine 2 (1996), 714-716; WO 94/29469; WO 97/00957; US 5,580,859; US 5,589,466; US 4,394,448 or Schaper, Current Opinion in Biotechnology 7 (1996), 635-640, and references cited therein. In particular, said vectors and/or gene delivery systems are also described in gene therapy approaches in neurological tissue/cells (see, inter alia Blömer, J. Virology 71 (1997) 6641-6649) or in the hypothalamus (see, inter alia, Geddes, Front Neuroendocrinol. 20 (1999), 296-316 or Geddes, Nat. Med. 3 (1997), 1402-1404). Further suitable gene therapy constructs for use in neurological cells/tissues are known in the art, for example in Meier (1999), J. Neuropathol. Exp. Neurol. 58, 1099-1110. The above described modes for introducing the vector into a desired target tissue or cell also find application if the introduction is for the purposes of gene therapy. Thus, the nucleic acid molecules and vectors may be designed for direct introduction or for introduction via liposomes, viral vectors (e.g. adenoviral, retroviral), electroporation, ballistic (e.g. gene gun) or other delivery systems into the cell. Additionally, a baculoviral system can be used as eukaryotic expression system for the nucleic acid molecules of the invention. The introduction and gene therapeutic approach should, preferably, lead to the expression of a functional compound.

As has been stated above, the method of the invention may be put into practice *in vivo, ex vivo* or *in vitro. In vivo* applications have been discussed above in the connection with gene therapy approaches. Ex vivo applications refer, *inter alia,* to embodiments wherein cells or tissues are treated outside the body in order to stimulate the proliferation of the mentioned cells in accordance with the invention and wherein said cells or tissues, upon successful stimulation may be reintroduced into an (experimental) non-human animal or into a human suffering from a related disease allowing the stimulation/treatment *in vivo* (for example, by way of an inducible promoter). *In vitro* applications include those that allow the further investigation of the mechanism by which proliferation is regulated including the identification of additional molecules in the regulation pathway.

In another preferred embodiment of the present invention, the promotion comprises introducing into said cells or neurons said (aa) (i) ICA512; or (ii) derivative thereof having ICA512 function; or (iii) fragment of ICA512 that may be cleaved by µ-calpain giving rise to a C-terminal fragment of ICA512 wherein said C-terminal fragment has the capability of being targeted to the nucleus; or (iv) derivative of said fragment of (iii) that may be cleaved by µ-calpain giving rise to a derivative of said C-terminal fragment of ICA512 wherein said derivative of said C-terminal fragment has the capability of being targeted to the nucleus; or (v) fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) fragment or derivative of ICA512 that may be cleaved by µ-calpain giving rise to a-C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally µ-calpain or a fragment or derivative thereof having µ-calpain function; or (b) said C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

In this preferred embodiment, the protein or peptidomimetic or other recited compound is directly introduced into the desired cell or tissue. Available processes for such an introduction include microinjection techniques or protein transduction procedures. See, for example, Lindsay MA. Peptide-mediated cell delivery: application in protein target validation. 2002. Curr Opin Pharmacol. 2:587-94.

An additional preferred embodiment of the present invention relates to a method wherein the promotion comprises reducing degradation or enhancing the stability in said cells or neurons of said (aa) (i) ICA512; or (ii) derivative thereof having ICA512 function; or (iii) fragment of ICA512 that may be cleaved by µ-calpain giving rise to a C-terminal fragment of ICA512 wherein said C-terminal fragment has the capability of being targeted to the nucleus; or (iv) derivative of said fragment of (iii) that may be cleaved by µ-calpain giving rise to a derivative of said C-terminal fragment of ICA512 wherein said derivative of said C-terminal fragment has the capability of being targeted to the nucleus; or (v) fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) pro-form of any one of (i) to (vi); and (ab) optionally µ-calpain or a fragment or derivative thereof having µ-calpain function; or (b) said C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

Reduction of the degradation comprises methods wherein proteins degrading any of the above recited proteinaceus compounds are inhibited such as by RNAi or antisense oligonucleotides. In an alternative, antibodies to said degrading proteins or antibody fragments or derivatives such as Fab, F(ab')₂ or scFv fragments may be employed; see, for example, Harlow and Lane, "Antibodies, A laboratory manual", CSH Press 1988, Cold Spring Harbor. The goal of this embodiment of the invention is to reduce unspecific degradation, not degradation by µ-calpain, which is, in contrast, desired in order to generate the preferred C-terminal fragment. Preferably, the enhancement and reduction, respectively, amount to at least 25% of the amount of the compounds, preferably at least 50%, more preferred at least 75% and most preferred at least 90%. In the case of enhancement of the stability, an increase of more than 100% is advantageously envisaged. One option to enhance stability is the generation of a peptidomimetic.

The C-terminal fragment to be employed in accordance with the present invention may be any fragment that retains the property of being targeted to the nucleus and promote expression of the genes encoding the mentioned neuropeptide. In addition or alternatively, the fragment maintains the capacity to interact with a PIAS protein and/or regulate the activity of transcription factors that promote expression of the genes encoding the mentioned neuropeptide. Tests for determining whether a fragment in question does have the required combination of properties may be carried out in accordance with the teachings of this specification without undue burden. Fragments may be designed and prepared according to conventional methods. They may be recombinantly produced, for example, by digestion of the coding region with an exonuclease from the N-terminus. Alternatively, a combination of site directed mutagenesis, restriction endonuclease digestion and ligation may be used to generate the desired fragment. In another alternative, either the (poly)peptide or the corresponding DNA encoding said (poly)peptide may be prepared (semi)synthetically using method known in the art to date. Other options to generate the desired fragment are available to the skilled artisan and include limited proteolytic digestion. In a preferred embodiment of the present invention, said C-terminal fragment of ICA512 consists of amino acids 659 to 979 of mature ICA512. This fragment is obtained e.g. by digestion with µ-calpain.

In another preferred embodiment of the present invention, said endocrine cells are pancreatic β-cells. As is well known in the art, type-2 diabetes goes along with an impaired production/secretion of insulin. This particularly preferred embodiment is thus expected to form a cure for type-2 diabetes.

It is further preferred that said promotion is effected by glucose or glucagon-like peptide-1 (GLP-1) or by calcium. Glucagon-like peptide-1 (GLP-1) is produced by intenstinal L-cells, which secrete it in response to food intake. The hormone affects the physiology of multiple organs, including pancreatic islets. Specifically, it rapidly potentiates insulin secretion and its biosynthesis. It also promotes islet cell growth and islet neogenesis. Most of these effects can be ascribed, directly or indirectly, to the elevation of intracellular cAMP levels, since binding of GLP-1 to its receptor stimulates adenylate cyclase activity See: MacDonald PE, El-Kholy W, Riedel MJ, Salapatek AM, Light PE, Wheeler MB. 2002. The multiple actions of GLP-1 on the process of glucose-stimulated insulin secretion. Diabetes 51 Suppl 3: 434-42. Calcium levels, i.e. the level of Ca⁺⁺ ions may advantageously be enhanced by ionophores or other compunds that directly or indirectly increase the opening probability and/or opening time of Ca²⁺ channels at the plasma membrane or by inhibiting the re-uptake of Ca²⁺ into intracellular stores, such as the cortical endoplasmic reticulum. This can be accomplished by inhibiting the Ca²⁺-pumps.

In a further preferred embodiment of the present invention, the peptide hormone or neuropeptide contained in said secretory granules is insulin, amylin or a peptide hormone or neuropeptide derived from one of the following precursors: ADM precursor, Agouti switch protein precursor, Agouti-related protein precursor, Apelin precursor, Atrial natriuretic factors, Beta-neoendorphin-dynorphin precursor, Brain natriuretic peptide precursor, Calcitonin gene-related peptide I precursor, Calcitonin gene-related peptide, II precursor, Calcitonin precursor, Cholecystokinin precursor, Chromogranin A precursor, Cocaine- and amphetamine-regulated transcript protein precursor, Corticoliberin precursor, Corticotropin-lipotropin precursor, Cortistatin precursor, FMRFamide-related peptides precursor, FMRFamide-related peptides precursor, Follistatin precursor, Follitropin beta chain precursor, Galanin precursor, Galanin-like peptide precursor, Gastric inhibitory polypeptide precursor, Gastrin precursor, Gastrin-releasing peptide precursor, Ghrelin precursor, Glucagon precursor, Glycoprotein hormones alpha chain precursor, Growth hormone variant precursor, Insulin precursor, Insulin-like growth factor binding protein 3 precursor, Insulin-like peptide INSL6 precursor, Islet amyloid polypeptide precursor, Leydig insulin-like peptide precursor, Morphogenetic neuropeptide, Motilin precursor, Neurexophilin 2 precursor, Neurexophilin 3 precursor, Neurexophilin 4 precursor, Neuroendocrine protein 7B2 precursor, Neurokinin B precursor, Neuromedin B-32 precursor, Neuromedin U-25 precursor, Neuropeptide B precursor, Neuropeptide W precursor, Neuropeptide Y precursor, Neurotensin precursor, Nociceptin precursor, Orexin precursor, Oxytocin-neurophysin 1 precursor, Pancreatic hormone precursor, Parathyroid hormone precursor, Parathyroid hormone-related protein precursor, Peptide YY precursor, Pituitary adenylate cyclase activating polypeptide precursor, Proenkephalin A precursor, Progonadoliberin I precursor, Progonadoliberin II precursor, Prokineticin 2 precursor, Prolactin precursor, Pro-MCH precursor, Prorelaxin H1 precursor, Protachykinin 1 precursor, Protein-tyrosine phophatase-like N precursor, Receptor-type protein-tyrosine phophatase N2 precursor, Regulated endocrine specific protein 18 precursor, Resistin precursor, Resistin-like beta precursor, Secretin precursor, Secretorygranin I precursor, Secretorygranin II precursor, Secretorygranin III precursor, Somatoliberin precursor, Somatostatin precursor, Somatotropin precursor, Stanniocalcin 1 precursor, Stanniocalcin 2 precursor, Urocortin II precursor, Urocortin precursor, Vasoactive intestinal peptide precursor, Vasopressin-neurophysin 2-copeptin precursor. It is of note and well known in the art that various hormones or neuropeptides are derivable from the precursors cited above. Important examples of such precursors and hormones or neuropeptides derived therefrom include the following:

### 1) Glucacagon precursor (P01275)

MKSIYFVAGL FVMLVQGSWQ RSLQDTEEKS RSFSASQADP LSDPDQMNED
KRHSQGTFTS
DYSKYLDSRR AQDFVQWLMN TKRNRNNIAK RHDEFERHAE GTFTSDVSSY
LEGQAAKEFI AWLVKGRGRR DFPEEVAIVE ELGRRHADGS FSDEMNTILD
NLAARDFINW LIQTKITDRK

### Derived peptides:

a) Glicentin-related polypeptide (residues 21-50): RSLQDTEEKS RSFSASQADP LSDPDQMNED
b) Glucagon (residues 53-81): HSQGTFTS DYSKYLDSRR AQDFVQWLMN T
c) Glucagon-like peptide 1 (residues 98-127): HAE GTFTSDVSSY LEGQAAKEFI AWLVKGR
d) Glucagon-like peptide 2 (residues 146-178): HADGS FSDEMNTILD NLAARDFINW LIQTKITD

### 2) Corticoliberin precursor (P06850)

MRLPLLVSAG VLLVALLPCP PCRALLSRGP VPGARQAPQH PQPLDFFQPP
PQSEQPQQPQ
ARPVLLRMGE EYFLRLGNLN KSPAAPLSPA SSLLAGGSGS RPSPEQATAN
FFRVLLQQLL
LPRRSLDSPA ALAERGARNA LGGHQEAPER ERRSEEPPIS LDLTFHLLRE
VLEMARAEQL AQQAHSNRKL MEIIGK

### Derived peptide:

a) Corticoliberin (residues 154-194 ): SEEPPIS LDLTFHLLRE VLEMARAEQL AQQAHSNRKL MEII

### 3) Corticotropin-lipotropin precursor (P01189)

MPRSCCSRSG ALLLALLLQA SMEVRGWCLE SSQCQDLTTE SNLLECIRAC
KPDLSAETPM FPGNGDEQPL TENPRKYVMG HFRWDRFGRR NSSSSGSSGA
GQKREDVSAG EDCGPLPEGG PEPRSDGAKP GPREGKRSYS MEHFRWGKPV
GKKRRPVKVY PNGAEDESAE AFPLEFKREL TGQRLREGDG PDGPADDGAG
AQADLEHSLL VAAEKKDEGP YRMEHFRWGS PPKDKRYGGF MTSEKSQTPL
VTLFKNAIIK NAYKKGE

### Derived peptides:

a) NPP (residues 27-102): WCLE SSQCQDLTTE SNLLECIRAC KPDLSAETPM FPGNGDEQPL TENPRKYVMG HFRWDRFGRR NSSSSGSSGA GQ
b) Melanotropin gamma (residues 77-87): YVMG HFRWDRF
c) melanotropin alpha (residues 138-150): SYS MEHFRWGKPV
d) Corticotropin (residues 138-176): SYS MEHFRWGKPV GKKRRPVKVY PNGAEDESAE AFPLEF
e) Lipotropin beta (residues 179-267): EL TGQRLREGDG PDGPADDGAG AQADLEHSLL VAAEKKDEGP YRMEHFRWGS PPKDKRYGGF MTSEKSQTPL VTLFKNAIIK NAYKKGE
f) Lipotropin gamma (residues 179-234): EL TGQRLREGDG PDGPADDGAG AQADLEHSLL VAAEKKDEGP YRMEHFRWGS PPKD
g) Melanotropin beta (residues 217-234) : DEGP YRMEHFRWGS PPKD
h) Beta endorphin (residues 237-267): YGGF MTSEKSQTPL VTLFKNAIIK NAYKKGE
i) Met-enkephalin (residues 237-241): YGGF M

### 4) Orexin precursor (043612)

MNLPSTKVSW AAVTLLLLLL LLPPALLSSG AAAQPLPDCC RQKTCSCRLY
ELLHGAGNHA AGILTLGKRR SGPPGLQGRL QRLLQASGNH AAGILTMGRR
AGAEPAPRPC LGRRCSAPAA
ASVAPGGQSG I

### Derived peptides:

a) Orexin A (residues 34-66): QPLPDCC RQKTCSCRLY ELLHGAGNHA AGILTL
b) Orexin B (residues 70-97): R SGPPGLQGRL QRLLQASGNH AAGILTM

### 5) Pituitary adenylate cyclase activating polypeptide precursor (P18509)

MTMCSGARLA LLVYGIIMHS SVYSSPAAAG LRFPGIRPEE EAYGEDGNPL
PDFGGSEPPG
AGSPASAPRA AAAWYRPAGR RDVAHGILNE AYRKVLDQLS AGKHLQSLVA
RGVGGSLGGG
AGDDAEPLSK RHSDGIFTDS YSRYRKQMAV KKYLAAVLGK RYKQRVKNKG RRIAYL

a) PACAP-related peptide (residues 82-129): DVAHGILNE AYRKVLDQLS AGKHLQSLVA RGVGGSLGGG AGDDAEPLS
b) Pituitary adenylate cyclase activating peptide-27 (residues 132-158): AHSDGIFTDS YSRYRKQMAV KKYLAAVL
c) Pituitary adenylate cyclase activating peptide- 38 (residues 132-169): HSDGIFTDS YSRYRKQMAV KKYLAAVLGK RYKQRVKNK

### 6) Ghrelin precursor (Q9UBU3)

MPSPGTVCSL LLLGMLWLDL AMAGSSFLSP EHQRVQQRKE SKKPPAKLQP
RALAGWLRPE
DGGQAEGAED ELEVRFNAPF DVGIKLSGVQ YQQHSQALGK FLQDILWEEA
KEAPADK

### Derived peptide:

a) Ghrelin (residues 24-51): GSSFLSP EHQRVQQRKE SKKPPAKLQP R

On the basis of this embodiment, a number of cures to pertinent diseases are feasible; see for further guidance: Harrison's Principles of Internal Medicine. By Eugene Braunwald M.D. (Editor), Anthony S. Fauci M.D. (Editor), Dennis L. Kasper M.D. (Editor), Stephen L. Hauser M.D. (Editor), Dan L. Longo M.D. (Editor), J. Larry Jameson M.D. (Editor). McGraw-Hill Professional; 15th edition (February 16, 2001) ISBN: 0070072728; Williams Textbook of Endocrinology, by Robert Hardin Williams (Editor), P. Reed Larsen (Editor), Jean D. Wilson, Melmed Shlomo, Daniel W. Foster, Henry M. Kronenberg. W B Saunders; 10th edition (December 20, 2002) ISBN: 0721691846. Peptides and Non Peptides in Neuroendocrinology and Oncology: From Basic to Clinical Research, by E. E. Muller (Editor) Springer-Verlag Telos (August 2003) ISBN: 8847002958.

A comprehensive list of accession numbers associated with the above hormones, neuropeptides and precursors thereof is provided by Table 1. Further information about the hormones/peptides/precursors can be obtained from these sources. A list of diseases associated with the hormones/peptides/precursors is provided by Table 1. It is to be understood in accordance with the present invention, that the invention comprises the treatment of any of the diseases associated with any of these hormones/peptides/precursors by manipulating the compound(s) presence/activity as described for the treatment of type-1 and type-2 diabetes, sleeping disorders or depression elsewhere in this specification with the exception that different cells are targeted, if appropriate. The skilled artisan is well aware which type of cell is to be targeted for each disease; see also the above cited textbooks for further guidance.

The present invention also relates to a method of treating or preventing type-1 or type-2 diabetes comprising stimulating expression of insulin in pancreatic β-cells wherein said stimulation comprises the step of promoting the presence or activity in said β-cells of (aa) of (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally of µ-calpain or a fragment or derivative thereof having µ-calpain function; or (b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

Furthermore, the present invention relates to the use of (aa) (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or (vii) a pro-form of any one of (i) to (vi); and (ab) optionally of µ-calpain or a fragment or derivative thereof having µ-calpain function; or (b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons for the preparation of a pharmaceutical composition for treating or preventing type-1 or type-2 diabetes.
Formulations of compounds into pharmaceutical compositions, regimens of treatment and modes of administration have been discussed herein above in connection with the method of the invention and apply equally here.

In another preferred embodiment of the present invention, said PIAS protein is PIASy, PIAS1, PIAS3, PIASxα or PIASxβ.

The present invention also relates to a method of screening for an agent capable of stimulating expression of peptide hormones in peptide hormone-secreting endocrine cells or neurons comprising the step of (a) assessing expression of said peptide hormones in a test cell expressing (aaa) (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus and has the capability of interacting with a PIAS protein in said cells or neurons or/and has the capability of enhancing the nuclear levels, or tyrosine phosphorylation, or DNA binding activity of STATs in said cells or neurons; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus and has the capability of interacting with a PIAS protein in said cells or neurons or/and has the capability of enhancing the nuclear levels, or tyrosine phosphorylation, or DNA binding activity of STATs in said cells or neurons; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a pro-form of any one of (i) to (v); and (aab) µ-calpain or a fragment or derivative thereof having µ-calpain function; or (ab) a C-terminal fragment of ICA512 or a derivative thereof which has the capability of interacting with a PIAS protein or/and the capability of being targeted to the nucleus or of enhancing the nuclear levels, or tyrosine phosphorylation, or DNA binding activity of STATs in said cells or neurons; and (ac) a PIAS protein or a fragment or derivative thereof having PIAS function in the presence and optionally in the absence of a test compound, wherein a higher level of expression in the presence of the test compound is indicative of its capacity to function as a stimulator of expression.
In accordance with the screening method of the invention, various options of carrying out said method are available. If option (aaa) is preferred, then µ-calpain has to be added to the substrate in order to allow cleavage of the ICA512 protein or of the fragment that contains the cleavage site for µ-calpain. As a result, a cleavage product is furnished that enters the nucleus and is in the position of functionally interacting with PIAS and/or regulate the activity of transcription factors. Conditions will normally be provided by the cell which allow the cleavage and interaction to occur. If the cell does not naturally express the mentioned and required proteins/peptides, then they may be introduced into the cell, for example via transfection; see above. Alternatively, a C-terminal fragment which preferably consists of amino acids 659 to 979 of mature ICA512 that has the above recited function may be contacted with the test compound(s) in the absence of µ-calpain.

It is important to note that the C-terminal fragment may extend N-terminally beyond position 659 of mature ICA512.

Contacting of the cell in step with the test compound(s) will be done, for example, under conditions that allow the uptake of the one or more compounds into the cell. Alternatively, conditions are provided where the compounds are allowed to interact with the surface receptors of the plasma membrane. For both embodiments, the conditions may be the same. Appropriate conditions include physiological conditions such as incubation in physiological saline. If more than one compound is contacted with the cell and the number of compounds tests positive, it is advantageous to repeat the experiment by incubating the cell with only one compound at the time. In so far, a direct relation between the compound tested and its capability to stimulate the expression can be established. The inclusion of an assessment step in the absence of a test compound is useful as a control, in this and the following embodiments of the invention.

The assessment in step (a) can be established by a variety of means. For example, peptide hormone levels can be measured by radioimmunoassay or by ELISA.

Said method of screening can be accomplished in a high-throughput manner. Robotic equipment for that purpose is known in the art and available from a number of suppliers. Cells are normally grown in wells of plates containing arrays of 96, 384, 1536 or more wells. Transfer of the well-plates from incubators, addition of test compounds, optional washing steps as well determining the read-out is performed in an automated fashion without requiring user interference using hundreds of thousands to millions of compounds in days to weeks.

Once a compound of interest has been identified, it can be further developed into a pharmaceutical agent such as by reducing its toxicity, prolonging shelf life and so on.

At variance but also making use of the gist of the invention (and outlined with different embodiments herein above) the present invention additionally relates to a method of screening for an agent capable of promoting cell proliferation of peptide hormone-secreting endocrine cells or neurons comprising the step of (a) assessing expression of said peptide hormones in a test cell expressing (aaa) (i) ICA512; or (ii) a derivative thereof having ICA512 function; or (iii) a fragment of ICA512 that may be cleaved by µ-calpain giving rise to a C-terminal fragment of ICA512 wherein said C-terminal fragment has the capability of being targeted to the nucleus and has the capability of interacting with a PIAS protein in said cells or neurons or/and has the capability of enhancing the nuclear levels, or tyrosine phosphorylation, or DNA binding activity of STATs in said cells or neurons; or (iv) a derivative of said fragment of (iii) that may be cleaved by µ-calpain giving rise to a derivative of said C-terminal fragment of ICA512 wherein said derivative of said C-terminal fragment has the capability of being targeted to the nucleus and has the capability of interacting with a PIAS protein in said cells or neurons or/and has the capability of enhancing the nuclear levels, or tyrosine phosphorylation, or DNA binding activity of STATs in said cells or neurons; or (v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or (vi) a pro-form of any one of (i) to (v); and (aab) µ-calpain or a fragment or derivative thereof having µ-calpain function; or (ab) a C-terminal fragment of ICA512 or a derivative thereof which has the capability of interacting with a PIAS protein or/and the capability of being targeted to the nucleus or of enhancing the nuclear levels, or tyrosine phosphorylation, or DNA binding activity of STATs in said cells or neurons; and (ac) a PIAS protein or a fragment or derivative thereof having PIAS function in the presence and optionally in the absence of a test compound, wherein a higher level of expression in the presence of the test compound is indicative of its capacity to function as a stimulator of proliferation.

The present invention also relates to a method of screening for an agent capable of stimulating expression of peptide hormones in peptide hormone-secreting endocrine cells or neurons the step of assessing expression of said peptide hormones in a test cell expressing (a) a PIAS protein or a fragment or derivative thereof having PIAS function; or/and (b) a C-terminal fragment of ICA512 preferably consisting of amino acids 659 to 979 of mature ICA512 in the presence and optionally in the absence of a test compound, wherein a higher level of expression in the presence of the test compound is indicative of its capacity to function as a stimulator of expression.
This embodiment of the invention makes use of the surprising finding that PIAS and a C-terminal fragment of ICA512, either alone or in conjunction, is required for the stimulation of the expression of neuropeptides. In so far, the design recited for the preceding embodiment may be somewhat simplified wherein the present embodiment nevertheless allows for the unambiguous identification of compounds useful at least as lead compounds for the development of agents that can be administered to patients in need thereof.
Again and at variance but also making use of the gist of the invention (and outlined with different embodiments herein above) the present invention additionally relates to a method of screening for an agent capable of promoting cell proliferation of peptide hormone-secreting endocrine cells or neurons comprising the step of (a) assessing expression of said peptide hormones in a test cell expressing (aa) a PIAS protein or a fragment or derivative thereof having PIAS function; or/and (ab) a C-terminal fragment of ICA512 preferably consisting of amino acids 659 to 979 of mature ICA512 in the presence and optionally in the absence of a test compound, wherein a higher level of expression in the presence of the test compound is indicative of its capacity to function as a stimulator of cell proliferation.

The present invention also relates to a method of screening for an agent capable of stimulating expression of peptide hormones in peptide hormone-secreting endocrine cells or neurons comprising the step of assessing expression of said peptide hormones under the control of an inducible promoter in a non-human transgenic animal expressing (a) a PIAS protein or a fragment or derivative thereof having PIAS function; or/and (b) a C-terminal fragment of ICA512 preferably consisting of amino acids 659 to 979 of mature ICA512 in the presence and optionally in the absence of a test compound, upon induction of said promoter, wherein a higher level of expression in the presence of the test compound is indicative of its capacity to function as a stimulator of expression.
The term "inducible promoter" is well known in the art and denotes a promoter the activity of which can be influenced by external parameters such as upon the addition of a drug. The promoter may, for example, be tissue specific or may be functional only during specific phases of development.
Methods for the production of a transgenic non-human animal, for example transgenic mouse, comprise introduction of a gene/polynucleotide as recited above or targeting vector into a germ cell, an embryonic cell, stem cell or an egg or a cell derived therefrom. The non-human animal can be used in accordance with a screening method of the invention described herein. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe; see supra. A general method for making transgenic non-human animals is described in the art, see for example WO 94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62: 1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326: 292-295 (1987)), the D3 line (Doetschman et al., J. Embryol. Exp. Morph. 87: 27-45 (1985)), the CCE line (Robertson et al., Nature 323: 445-448 (1986)), the AK-7 line (Zhuang et al., Cell 77: 875-884 (1994) which is incorporated by reference herein). The success of generating a mouse line from ES cells bearing a specific targeted mutation depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant nonhuman females and are born as chimeric mice. The resultant transgenic mice are chimeric for cells having either the recombinase or reporter loci and are backcrossed and screened for the presence of the correctly targeted transgene (s) by PCR or Southern blot analysis on tail biopsy DNA of offspring so as to identify transgenic mice heterozygous for either the recombinase or reporter locus/loci.
Methods for producing transgenic flies, such as Drosophila melanogaster are also described in the art, see for example US-A-4,670,388, Brand & Perrimon, Development (1993) 118: 401-415; and Phelps & Brand, Methods (April 1998) 14: 367-379. Transgenic worms such as C. elegans can be generated as described in Mello, et al., (1991) Efficient gene transfer in C.elegans: extrachromosomal maintenance and integration of transforming sequences. Embo J 10, 3959-70, Plasterk, (1995) Reverse genetics: from gene sequence to mutant worm. Methods Cell Biol 48, 59-80.
The transgenic animal may be contacted with the test compounds, for example by ingestion with the food or by injection, either into the blood stream or into the site where the recited polynucleotides are expressed upon the induction of the promoter.

Also, the present invention relates to a method of screening for an agent capable of promoting cell proliferation of peptide hormone-secreting endocrine cells or neurons comprising the step of assessing expression of said peptide hormones under the control of an inducible promoter in a non-human transgenic animal expressing (a) a PIAS protein or a fragment or derivative thereof having PIAS function; or (b) a C-terminal fragment of ICA512 preferably consisting of amino acids 659 to 979 of mature ICA512 in the presence and optionally in the absence of a test compound, upon induction of said promoter, wherein a higher level of expression in the presence of the test compound is indicative of its capacity to function as a stimulator of proliferation.

As stated above, the cell employed may be a cell that naturally expresses the required proteins/peptides (not the test compounds) or a cell that does not. In a preferred embodiment of the present invention, said test cell is a hormone-secreting endocrine cell or a neuron.

In a most preferred embodiment of the present invention, said endocrine cells are pancreatic β-cells.
Pancreatic β-cells are expected to be most useful in the development of cures for type-2 diabetes.

In another particularly preferred embodiment of the present invention, the secretory-peptide contained in said secretory granules is insulin.

The present invention also relates to a method of screening for an agent capable of stimulating expression of insulin in pancreatic β-cells comprising the step of assessing expression of insulin in a test cell expressing (a) a PIAS protein or a fragment or derivative thereof having PIAS function; or/and (b) a C-terminal fragment of ICA512 preferably consisting of amino acids 659 to 979 of mature ICA512 in the presence and optionally in the absence of a test compound, wherein a higher level of expression in the presence of the test compound is indicative of its capacity to function as a stimulator of insulin expression.

Further, the present invention also relates to a method of screening for an agent capable of stimulating proliferation of pancreatic β-cells comprising the step of assessing expression of insulin in a test cell expressing (a) a PIAS protein or a fragment or derivative thereof having PIAS function; or (b) a C-terminal fragment of ICA512 preferably consisting of amino acids 659 to 979 of mature ICA512 in the presence and optionally in the absence of a test compound, wherein a higher level of expression in the presence of the test compound is indicative of its capacity to function as a stimulator of proliferation.

In a preferred embodiment of the present invention, said compound is a member of a library of compounds.

In a further particularly preferred embodiment of the present invention, said library of compounds is a library of small molecules, peptides, antibodies or antibody derivatives, such as Fabs, F(ab₂)', scFvs, humanized or chimeric antibodies.

In any of the above mentioned methods or uses of the present invention, it is preferred that the derivative (of the various compounds) is a mimetic and preferably a peptidomimetic, a peptide aptamer or an anticalin. Peptide aptamers are small molecules drugs that inhibit intracellular protein interactions (Hoppe-Seyler F, Crnkovic-Mertens I, Tomai E, Butz K. 2004. Peptide aptamers: specific inhibitors of protein function. Curr Mol Med. 2004 Aug;4(5):529-38). Anticalins are engineered proteins with antibody-like ligand-binding functions derived from natural lipocalins as a scaffold (Skerra A. 2001 'Anticalins': a new class of engineered ligand-binding proteins with antibody-like properties. J Biotechnol. 74:257-75)
Consequently, in this and other embodiments pertaining to screening methods, the agent to be screened can be contained in libraries of small molecules, such as organic or inorganic small molecules which may be commercially available. In addition, libraries comprising antibodies or functional fragments or derivatives thereof (i.e. fragments or derivatives maintaining the binding specificity of the original antibody) may be used as a starting point in the screening process. Also, libraries of aptamers such as peptide aptamers might be employed. The skilled artisan is of course free to use any other starting point of desired compounds for use in the screen assays described throughout the specification. Suitable libraries are commercially available, for example from ChemBridge Corp., San Diego, USA.

Preferred in accordance with the present invention is further a method that further comprises the step of (c) testing the efficacy of a compound assessed as being capable of stimulating the expression of said peptide hormones or promoting cell proliferation of peptide hormone secreting endocrine cells or neurons in an animal model.
This additional step will allow significant insights in the in vivo applicability of the compound that tested positive in an in vitro assay. Parameters like toxicity, half-life etc. that have to be tested in pre-clinical trials may be assessed in this step. Suitable (non-human) animals foremost include laboratory mice and rats such as available from Charles River Laboratories etc.

Additionally, the invention envisages that the method comprises the further step of improvement or of refining the pharmacological properties of the identified agent (in the following also referred to as "compound" or "drug"), by the method as described herein above, said method comprising the optionally the steps of said methods and:
(1) identification of the binding sites of the compound and interacting protein by site-directed mutagenesis or chimeric protein studies;
(2) molecular modeling of both the binding site of the compound and the binding site of the interacting protein; and
(3) modification of the compound to improve its binding specificity for the interacting protein.

All techniques employed in the various steps of the method of the invention are conventional or can be derived by the person skilled in the art from conventional techniques without further ado. Thus, biological assays based on the herein identified nature of the interacting protein may be employed to assess the specificity or potency of the drugs wherein the increase of one or more activities of the interacting protein may be used to monitor said specificity or potency. Steps (1) and (2) can be carried out according to conventional protocols. A protocol for site directed mutagenesis is described in Ling MM, Robinson BH. (1997) Anal. Biochem. 254: 157-178. The use of homology modeling in conjunction with site-directed mutagenesis for analysis of structure-function relationships is reviewed in Szklarz and Halpert (1997) Life Sci. 61:2507-2520. Chimeric proteins are generated by ligation of the corresponding DNA fragments via a unique restriction site using the conventional cloning techniques described in Sambrook (1989), loc. cit.. A fusion of two DNA fragments that results in a chimeric DNA fragment encoding a chimeric protein can also be generated using the gateway-system (Life technologies), a system that is based on DNA fusion by recombination. A prominent example of molecular modeling is the structure-based design of compounds binding to HIV reverse transcriptase that is reviewed in Mao, Sudbeck, Venkatachalam and Uckun (2000). Biochem. Pharmacol. 60: 1251-1265.
For example, identification of the binding site of said drug by site-directed mutagenesis and chimerical protein studies can be achieved by modifications in the primary sequence of the interacting protein that affect the drug affinity; this usually allows to precisely map the binding pocket for the drug.
As regards step (2), the following protocols may be envisaged: Once the effector site for drugs has been mapped, the precise residues interacting with different parts of the drug can be identified by combination of the information obtained from mutagenesis studies (step (1)) and computer simulations of the structure of the binding site provided that the precise three-dimensional structure of the drug is known (if not, it can be predicted by computational simulation). If said drug is itself a peptide, it can be also mutated to determine which residues interact with other residues in the (poly)peptide of interest.
Finally, in step (3) the drug can be modified to improve its binding affinity or its potency and specificity. If, for instance, there are electrostatic interactions between a particular residue of the interacting protein of interest and some region of the drug molecule, the overall charge in that region can be modified to increase that particular interaction.
Identification of binding sites may be assisted by computer programs. Thus, appropriate computer programs can be used for the identification of interactive sites of a putative inhibitor and the (poly)peptide by computer assisted searches for complementary structural motifs (Fassina, Immunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. Modifications of the drug can be produced, for example, by peptidomimetics and other inhibitors can also be identified by the synthesis of peptidomimetic combinatorial libraries through successive chemical modification and testing the resulting compounds. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of activators of the expression of PTB can be used for the design of peptidomimetic activators (Rose, Biochemistry 35 (1996), 12933-12944; Rutenber, Bioorg. Med. Chem. 4 (1996), 1545-1558).

In accordance with the above, in a preferred embodiment of the method of the invention said pharmacological properties of the identified inhibitor or antagonist is further improved or refined by peptidomimetics.

The method of the invention can further include modifying a compound identified, improved of refined by the method as described herein above as a lead compound to achieve (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmakinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carbon acids, or (iii) esterification of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophylic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiozolidinesor combinations thereof; said method optionally further comprising the steps of the above described methods.
The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

The invention moreover contemplates in another preferred embodiment a method wherein the compound identified and optionally further modified as indicated above is formulated into a pharmaceutical composition.

### The figures show:

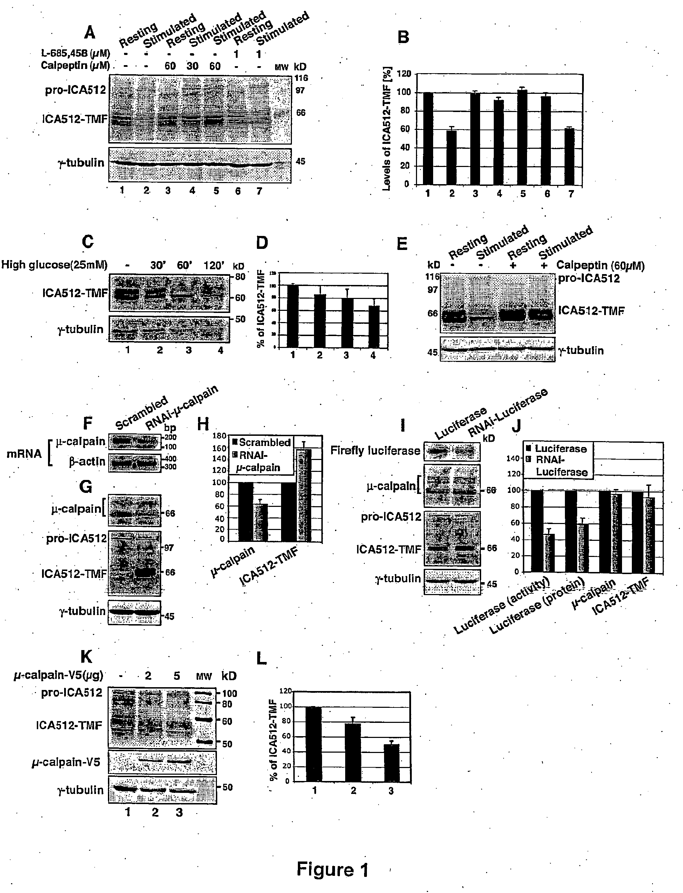

**Figure 1.** ICA512 cytoplasmic domain is cleaved by µ-calpain in response to stimulation of β-cells. **(A)** Western blot for ICA512 (top panel) and γ-tubulin (bottom panel) on 40µg protein from INS-1 cells which had been incubated with resting or stimulating buffer for 105 min. following 1 hr. at rest. Protease inhibitors L-685,458 and calpeptin were added at the indicated concentrations. **(B)** Quantitation of ICA512-TMF from 4 independent experiments as shown in (A). **(C)** Western blots for ICA512 (top panel) and γ-tubulin (bottom panel) on 30µg protein from rat islets which were incubated with 0 mM (resting) or 25 mM (stimulated) glucose for the indicated times. **(D)** Quantitation of ICA512-TMF from 2 independent experiments as shown in (C)**. (E)** Western blots for ICA512 (top panel) and γ-tubulin (bottom panel) on 30µg protein from rat islets which were incubated in resting or stimulating buffers as in (A), with or without 60 µM calpeptin. **(F)** Levels of µ-calpain and β-actin mRNAs by semiquantitative RT-PCR in INS-1 cells transfected with scrambled or anti-µ-calpain siRNA oligos 1. **(G)** Western blots for µ-calpain (top panel), ICA512 (middle panel) and γ-tubulin (bottom panel) on 40µg protein fromstimulated INS-1 cells transfected with scrambled or anti-µ-calpain siRNA oligos 1. **(H)** Quantitation of µ-calpain and ICA512-TMF from 3 independent experiments as shown in (G). **(I)** Western blots for firefly luciferase (top panel); µ-calpain (middle top panel), ICA512-TMF (middle bottom panel) and γ-tubulin (bottom panel) on 30µg protein in INS-1 cells transfected with firefly luciferase cDNA and then untreated or treated with siRNA oligos for firefly luciferase. **(J)** Firefly luciferase activity and protein levels of firefly luciferase, µ-calpain and ICA512-TMF from 3 independent experiments as shown in (I). **(K)** Western blots for ICA512 (top panel), µ-calpain-V5 (middle panel) and γ-tubulin (bottom panel) on 40µg protein from INS-1 cells transfected with the indicated amount of cDNA for µ-calpain-V5 and stimulated as in (A) **(L)** Quantitation of ICA512-TMF from 3 independent experiments as shown in (K). In (B), (D), (H), (J) and (L) protein signals were normalized for γ-tubulin and expressed as a percentage of their respective values in cells at rest (B and D), transfected with scrambled siRNA oligos (H) or firefly luciferase cDNA (J), or electroporated (L). Error bars in (B), (D), (H), (J) and (L) show mean + SD.

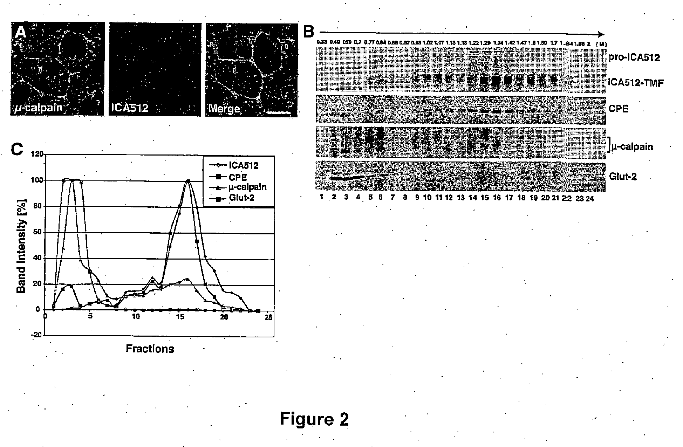

**Figure 2.** µ-Calpain is enriched at the plasma membrane. **(A)** Confocal microscopy of INS-1 cells double immunostained with anti-µ-calpain (pseudogreen) and anti-ICA512ecto (pseudored) antibodies. Bar: 10 µm. **(B)** Western blottings with anti-µ-calpain, anti-ICA512, anti-CPE and anti-Glut2 antibodies on INS-1 cell fractions separated on a continuous sucrose density gradient. The sucrose molarity of each fraction is indicated on the top. **(C)** Protein distribution in the fractions shown in (B). The highest signal for each protein was equaled to 100%.

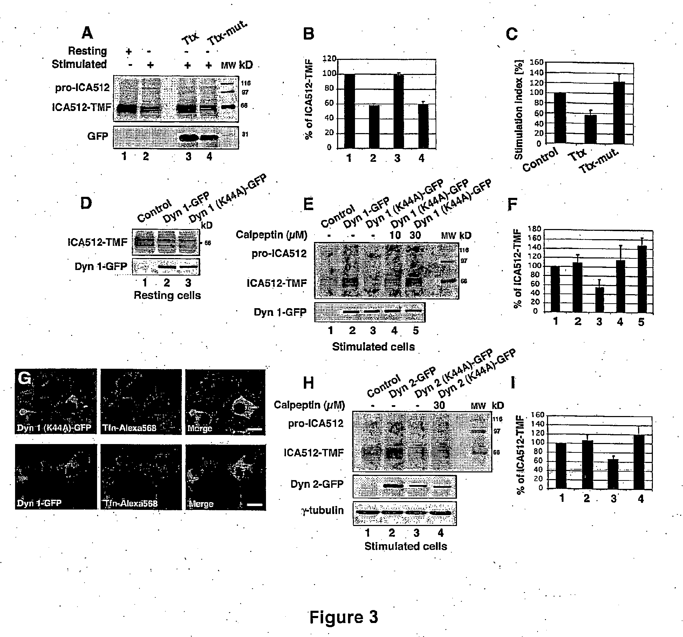

**Figure 3.** ICA512 is cleaved by µ-calpain at the plasma membrane. **(A)** Western blottings with anti-ICA512 (top) or GFP (bottom) antibodies on 40µg protein from INS-1 cells co-transfected with active (Ttx) or inactive (Ttx-mut.) tetanus toxin light chain and GFP. Cells were incubated in resting or stimulating buffer for 105 min. following 1 hr. at rest. **(B)** Quantitation of ICA512-TMF from 3 independent experiments as shown in (A). **(C)** Stimulation index of insulin secretion from INS-1 cells which were either non-transfected or transfected with Ttx or Ttx-mut. **(D)** Western blotting with anti-ICA512 (top) and anti-GFP (bottom) antibodies on 30µg protein from INS-1 cells, which were either non-transfected (control) or transiently transfected with dynamin 1-GFP or dynamin 1 (K44A)-GFP and kept in resting buffer for 1 hr. **(E)** and **(H)** Western blotting with anti-ICA512 (top) and anti-GFP (bottom) antibodies on 20µg protein from INS-1 cells which were either non-transfected (control) or transiently transfected in (E) with dynamin 1-GFP or dynamin 1 (K44A)-GFP, and in (H) with dynamin 2-GFP or dynamin 1 (K44A)-GFP. Cells were stimulated for 105 min. in the absence or presence of calpeptin at the indicated concentration. As the expression of dynamin 2 constructs differed, loading of comparable protein amount was verified by immunoblot for γ-tubulin. **(F)** and **(I)** Quantitation of ICA512-TMF from 4 independent experiments as shown in (E) and (H), respectively. Error bars in (B), (C), (F) and (I) show mean + SD. **(G)** Fluorescence microscopy on INS-1 cells transiently transfected with dynamin 1 (K44A)-GFP (top) or dynamin 1-GFP (bottom) and incubated with Alexa⁵⁶⁸-transferrin (Tfn-Alexa568). Bars: 10 µm.

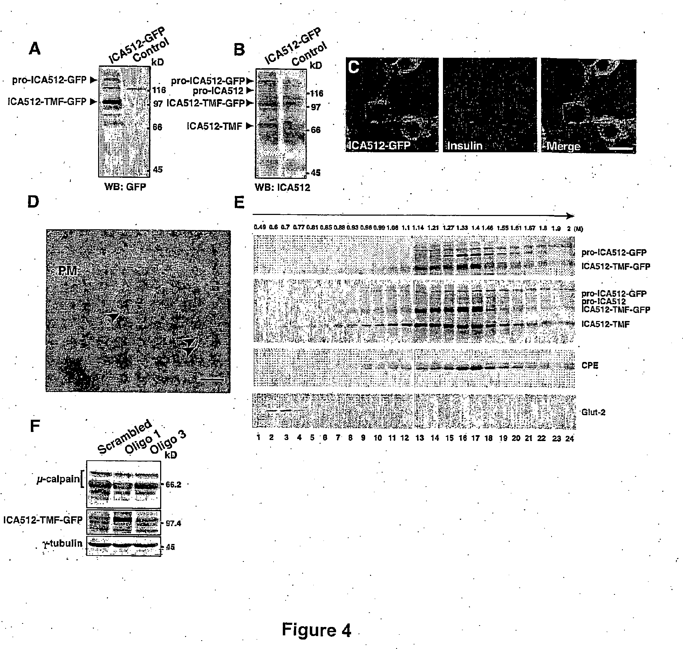

**Figure 4.** ICA512-GFP is correctly processed and targeted to SGs. **(A)** and **(B)** Western blottings with anti-GFP (A) or anti-ICA512 (B) antibodies on 40µg protein from INS-1 cells non-transfected (control) or stably transfected with ICA512-GFP. **(C)** Confocal microscopy on INS-1 ICA512-GFP cells immunostained for insulin (pseudored). Bar: 10 µm. **(D)** Immunoelectronmicroscopy on resting INS-1 ICA512-GFP cells double labeled with anti-insulin (6nm gold) and anti-GFP (12nm gold, arrowheads) antibodies. PM: plasma membrane. Bar: 200nm. **(E)** Western blottings with anti-GFP, anti-ICA512, anti-CPE and anti-Glut2 antibodies on fractions of INS-1 ICA512-GFP cells separated on a continuous sucrose density gradient. The sucrose molarity of each fraction is indicated on the top. **(F)** Western blots for µ-calpain (top), ICA512 (middle) and γ-tubulin (bottom) on 40µg protein from INS-1 cells transfected with scrambled or anti-µ-calpain siRNA oligos 1 or 3.

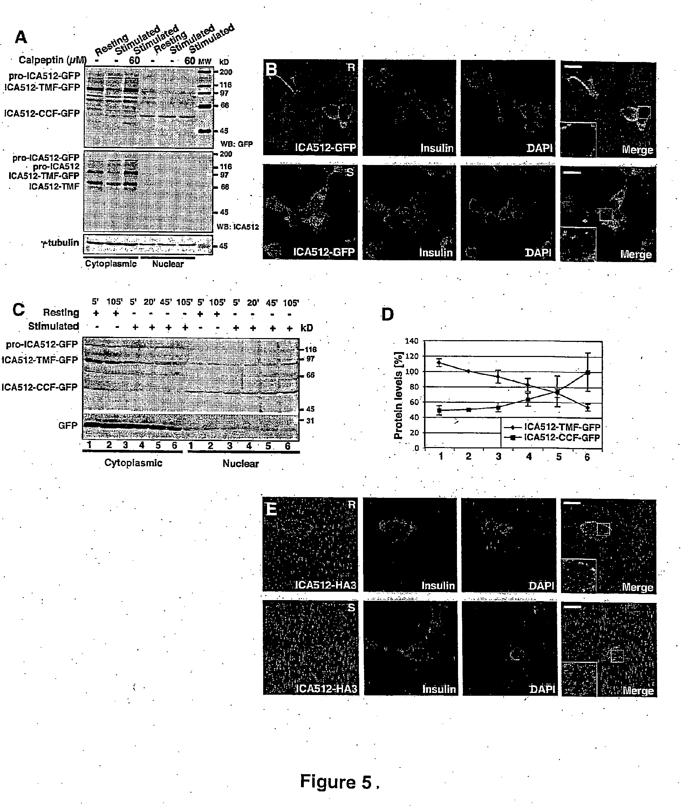

**Figure 5.** ICA512-CCF is translocated to the nucleus upon stimulation. **(A)** Western blots with anti-GFP (top), anti-ICA512 (middle) and γ-tubulin (bottom) on 50µg cytoplasmic and 25µg nuclear protein from INS-1 ICA512-GFP cells. Cells were incubated with resting or stimulating buffer for 105 min. after 1 hr at rest and with or without 60µM calpeptin. **(B)** 0.5 µm optical Z-sections of INS-1 ICA512-GFP cells kept at rest (R, top) or stimulated (S, bottom) as in (A) and immunolabeled with anti-insulin antibody (pseudored). ICA512-GFP is shown as pseudogreen, while nuclei were counterstained with DAPI (pseudoblue). Insets in the right panels show high magnifications of the marked areas. **(C)** Western blots with anti-GFP on 40µg cytoplasmic and 20µg nuclear protein from INS-1 ICA512-GFP cells incubated as in (A) for the indicated times. **(D)** Quantitation of ICA512-TMF-GFP and ICA512-CCF-GFP in cytoplasmic (black line) and nuclear (red line) extracts at the indicated time points 1-6. Values are from 4 independent experiments as shown in (C). For graphic representation, the amounts of ICA512-TMF-GFP and ICA512-CCF-GFP after 105 minutes in resting buffer (time point 2) were equaled to 100% and 50%, respectively. Error bars show mean ± SD. **(E)** 0.5µm optical Z-sections of INS-1 cells transiently transfected with ICA512-HA, kept at rest (R, top) or stimulated (S, bottom) as in (A), and immunolabeled with rabbit anti-HA (pseudogreen), and mouse anti-insulin (pseudored) antibodies. Nuclei were counterstained with DAPI (pseudoblue). Insets in the right panels show high magnifications of the marked areas. Bars in (B) and (E): 10 µm.

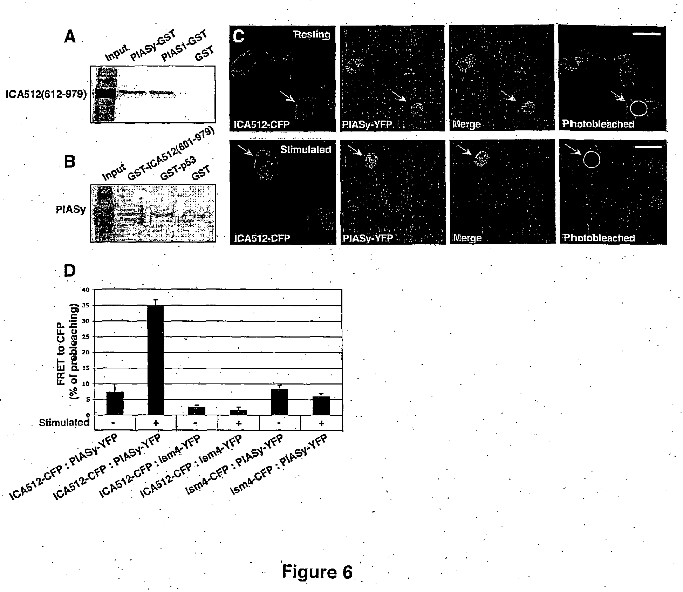

**Figure 6.** ICA512-CCF binds PIASy in the nucleus. **(A)** SDS-PAGE and autoradiography of in-vitro transcribed and translated ³⁵S-methionine-labeled ICA512(601-979) pulled down with glutathione sepharose beads coupled to 0.5µg PIASy-GST, PIAS1-GST or GST alone. Lane 1 shows the starting labeled material for the pull-down assay. **(B)** SDS-PAGE and autoradiography of in-vitro transcribed and translated ³⁵S-methionine-labeled PIASy pulled down with glutathione sepharose beads coupled to 0.5 µg GST-ICA512(601-979), GST-p53 or GST alone. Lane 1 shows the starting labeled material for the pull-down assay. **(C)** FRET between ICA512-CFP (pseudocyan) and PIASγ-YFP (pseudogreen) in transiently co-transfected INS-1 cells incubated in resting or stimulating buffer for 105 min. after 1hr at rest. The right panels show the signal for ICA512-CFP following the photobleaching of PIASy-YFP in the circled nuclei (arrows). **(D)** Quantitation of FRET to CFP in INS-1 cells transiently co-transfected with the indicated constructs: ICA512-CFP + PIASy-YFP; ICA512-CFP + Ism4-YFP; lsm4-CFP + PIASy-YFP. Values are from 2 independent experiments as shown in (C). Error bars shown in (D) show mean + SD.

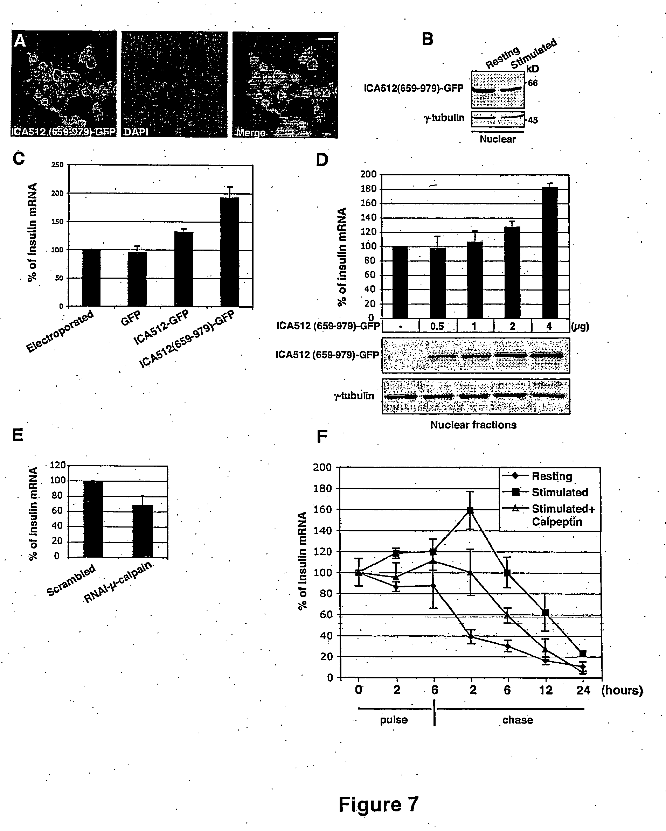

**Figure 7. (A)** Confocal microscopy on INS-1 cells transiently transfected with ICA512 (659-979)-GFP and counterstained with DAPI. Bar: 10 µm **(B)** Western blot with goat anti-GFP (top) and anti-γ-tubulin (bottom) antibodies on 6µg nuclear protein from INS-1 cells transiently transfected with ICA512(659-979)-GFP and incubated with resting or stimulating buffer for 105 min. after 1 hr. at rest. **(C)** Insulin mRNA levels quantified by real-time PCR in INS-1 cells which were either electroporated only or transfected with 4µg vector encoding GFP, ICA512-GFP or ICA512(659-979)-GFP and kept at rest for 1 hr. The results are from 3 independent experiments, each in triplicate. **(D)** Insulin mRNA levels quantified by real-time PCR in INS-1 cells which were either electroporated only or transfected with 0.5-4µg ICA512(659-979)-GFP and collected after 4 days in culture with 11 mM glucose. The results are from 4 independent experiments, each in triplicate. In (C) and (D) the levels of insulin mRNA in electroporated cells were equaled to 100%. In (D) ICA512(659-979)-GFP and γ-tubulin levels were assessed by western blotting with goat anti-GFP (upper panel) and anti-γ-tubulin (lower panel) antibodies. **(E)** Insulin mRNA levels quantified by real-time PCR in INS-1 cells transfected either with scrambled or anti-µ-calpain siRNA oligos 1 + 2. The results are from 3 independent experiments, each in triplicate. The level of insulin mRNA in cells transfected with the scrambled oligos was equaled to 100%. Error bars shown in (C), (D) and (E) show mean + SD. (F) Insulin mRNA levels quantified by real-time PCR in INS-1 cells which were at rest (2.8 mM glucose and 5 mM KCI) for 1 hr., then in resting or stimulating buffer, with or without 40 µM calpeptin, for up to 6 hrs. (pulse) and finally in resting buffer for up to 24 hrs (chase). Total RNA was collected at 0, 2 and 6 hr. time points of the pulse and then at 2, 6, 12 and 24 hr. time points of the chase. The results are from 3 independent experiments, each in triplicate. The level of insulin mRNA in resting INS-1 cells was equaled to 100%. Error bars show mean ± SD.

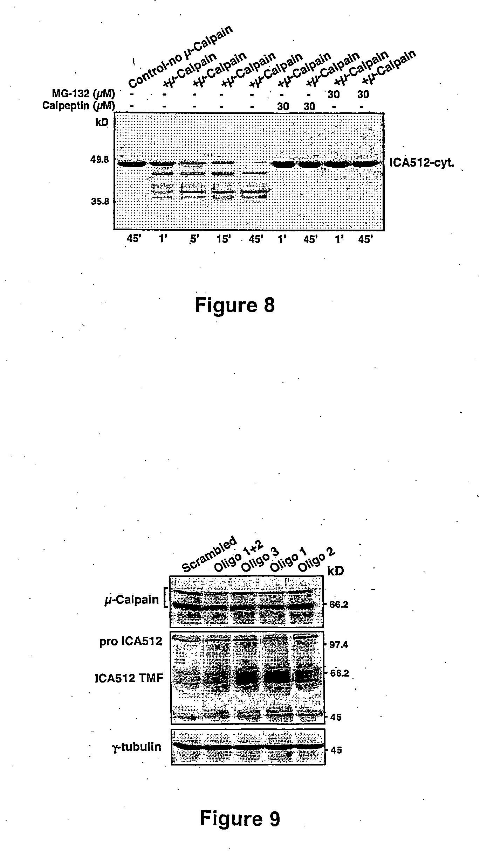

**Figure 8**. Coomassie Blue staining of a 10% SDS-polyacrylamide gel loaded with bacterially expressed ICA512 (601-979) incubated at room temperature in 50µM Ca²⁺ containing buffer with or without µ-calpain and 30µM calpeptin or MG-132 for the indicated time periods.
**Figure 9**. Western blots for ICA512 (top panel), µ-calpain-V5 (middle panel) and γ-tubulin (bottom panel) on 40µg protein extracts from INS-1 cells transfected with construct encoding µ-calpain-V5 at the indicated concentrations, which were incubated with resting (0 mM glucose, 5 mM KCI) or stimulating (25 mM glucose, 55 mM KCI) buffer with or without calpeptin for 105 min. after 60 min. in resting buffer.

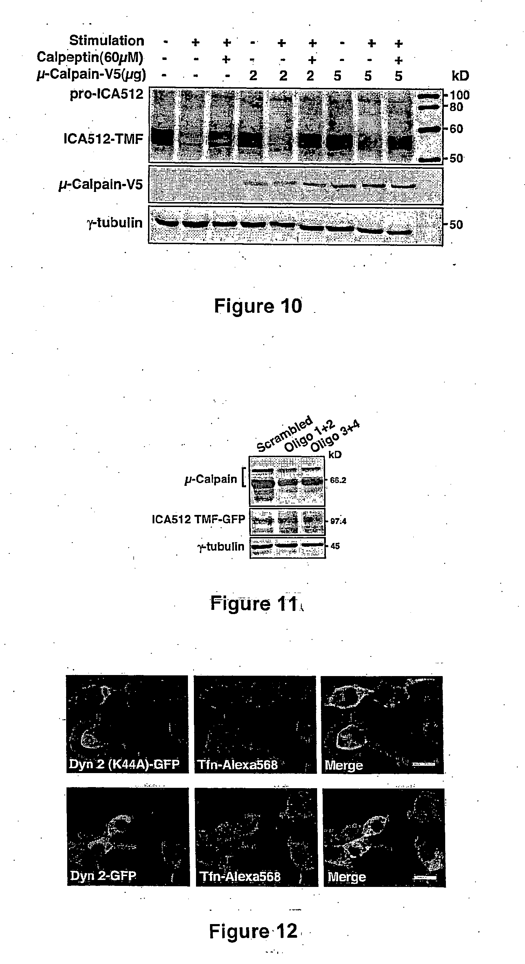

**Figure 10.** Western blots for µ-calpain (top panel), ICA512 (middle panel) and γ-tubulin (bottom panel) on 40µg protein extracts from INS-1 cells transfected with scrambled or anti-µ-calpain siRNA oligos 1+2, 3, 1 or 2.
**Figure 11.** Western blots for µ-calpain (top panel), GFP (middle panel) and γ-tubulin (bottom panel) on 30µg protein extracts from ICA512 INS-1 cells transfected with scrambled or anti-µ-calpain siRNA oligos 1+2 or 3+4.

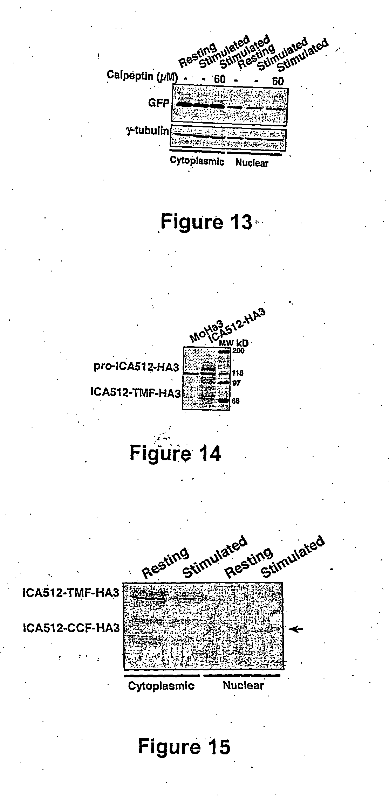

**Figure 12.** Fluorescence microscopy on INS-1 cells transiently transfected with dynamin 2 (K44A)-GFP (top) or dynamin 2-GFP (bottom) and incubated with human transferrin conjugated to Alexa⁵⁶⁸ (Tfn-Alexa⁵⁶⁸). Bars: 10 µm.
**Figure 13.** Western blots with anti-GFP (top) or anti-γ-tubulin (bottom) antibodies on 50µg cytoplasmic and 25µg nuclear protein extracts from INS-1 cells transfected with GFP, and incubated in resting or stimulating conditions with or without calpeptin as in figure S2.
**Figure 14.** Western blot with mouse anti-HA antibody on 40µg protein extracts from INS-1 cells transfected with MoHa3 vector or ICA512-HA3.
**Figure 15.** Western blot with mouse anti-HA antibody on 14µg cytoplasmic and 7µg nuclear protein extracts from INS-1 cells transiently transfected with ICA512-HA3 and kept at rest or stimulated as in figure S2.

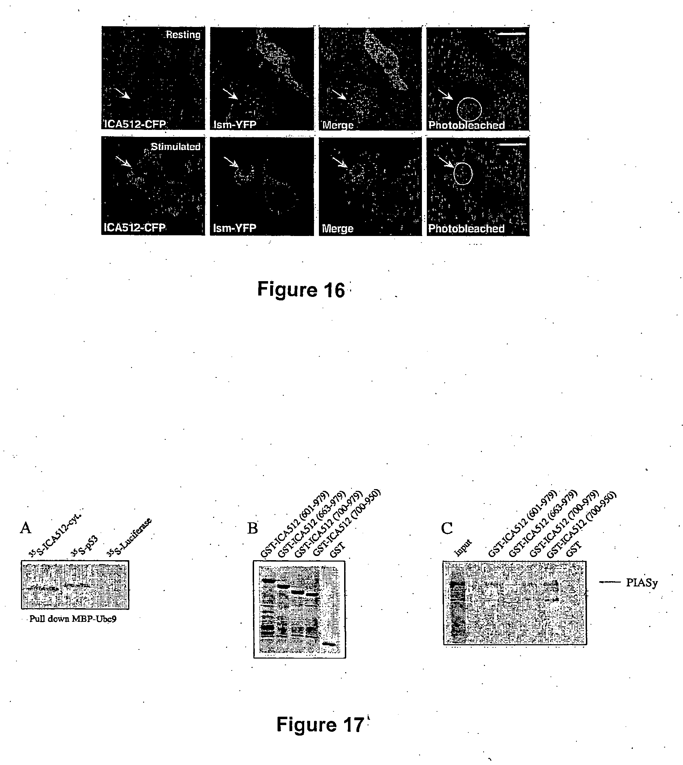

**Figure 16.** No FRET between ICA512-CFP (pseudocyan) and lsm4-YFP (pseudogreen) in transiently co-transfected INS-1 cells incubated in resting (top) or stimulating (bottom) as in figure S2. The right panels show the absence of signal for ICA512-CFP following the photobleaching of Ism4-YFP in the circled nuclei (arrows). Bars: 5 µm.
**Figure 17.** ICA512 binds the E2-SUMO ligase Ubc9 and its PTP domain is required for the interaction with PIASy.
   **(A)**, [35S]-labeled IVTT ICA512 cytoplasmic domain (ICA512-cyt), p53 as positive and luciferase as negative controls, respectively were independently incubated with recombinant Ubc9 fused to maltose binding protein (MBP-Ubc9). Proteins were pulled down with amylose resin. The beads were washed and eluted. The supernatant was subjected to SDS-PAGE and revealed by autoradiography. **(B)**, Ponceau red staining of four purified GST-ICA512 constructs expressed as recombinant proteins in bacteria. **(C)**, Pull down assay and autoradiography with GST-ICA512 constructs incubated with [35S-labeled IVTT PIASy.

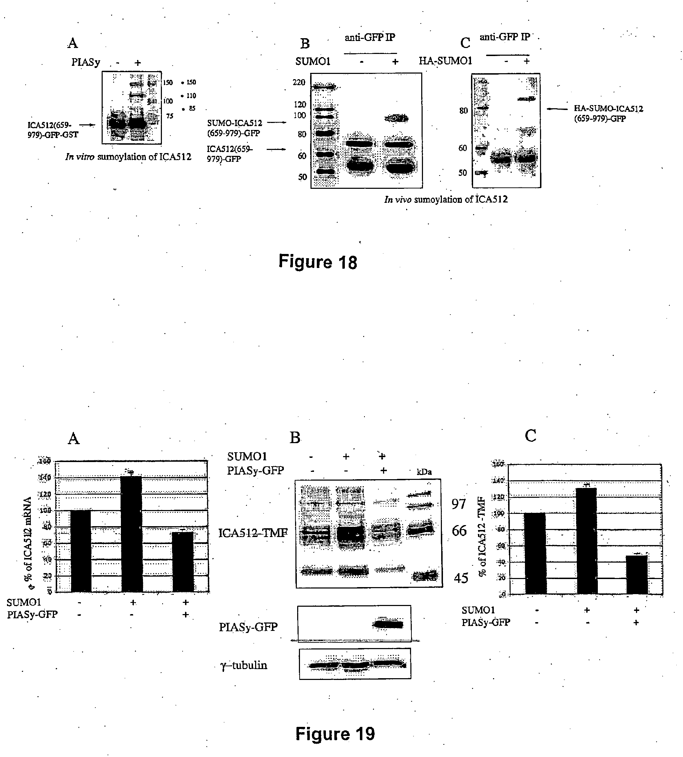

**Figure 18.** ICA512 is sumoylated in vitro and in vivo.
   **(A),** ICA512 cyt. (601-979) was expressed in bacteria as GST fusion protein and incubated in vitro with the sumoylation enzymatic machinery containing E1 (heterodimer Aos1/Uba2), Ubc9, and SUMO1 Where indicated, 100 ng of GST-PIASy was added. The sumoylation reaction was subjected to SDS-PAGE and revealed by western blot with the monoclonal antibody mICA512. Asterisks indicate the sumoylated forms of ICA512. **(B and C)**, ICA512 is SUMO-modified in vivo. ICA512(659-979)-GFP was expressed in INS-1 cells alone or co-expressed either with SUMO1 (B) or HA-SUMO1 (C). Equal amount of total cellular protein was immunoprecipitated with anti-GFP Ab. Western blots were performed with anti-GFP Ab (B), or an anti-HA Ab (C).
**Figure 19.** ICA512 expression is sensitive to SUMO1 and PIASy. (A), INS-1 cells were transfected either with an empty vector, with SUMO1 or SUMO1 plus PIASy-GFP. ICA512 mRNA from transfected INS-1 cells was quantified by a real time PCR. (B), INS-1 cells were transfected as in (A) and total cell lysates were analyzed by western blotting with anti-ICA512 and anti-GFP antibodies. The blot was also probed with an anti-γ-tubulin Ab to check for protein loading. (C), The levels of ICA512-TMF were quantified using Image Quant (Fuji, Inc.) and normalized with the signal for γ-tubulin band. The values in (A) and (C) are from two independent experiments.

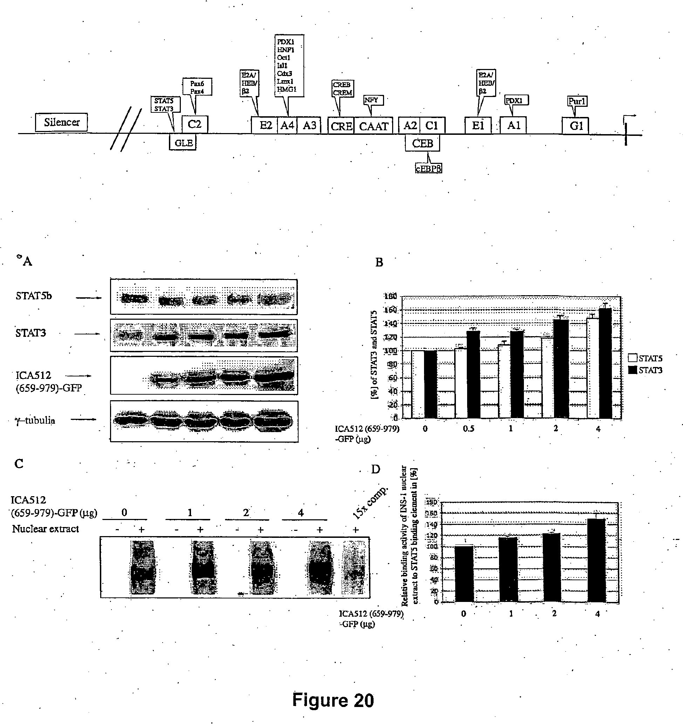

**Figure 20. (top)** Organization of rat I insulin enhancer and promoter region. Cis-acting regulatory elements along the promoter regions of these genes are boxed. Proteins binding to these sequences are indicated above each box.
   **(bottom)** ICA512 affects STAT3 and STAT5 nuclear localization and enhances the binding of STAT5 to GLE, a STAT5 binding element. (A and B), Western blot (A) and corresponding quantitative analyses (B) demonstrating that overexpression of ICA512 (659-979)-GFP correlates with an accumulation of STAT5b and STAT3 in the nucleus in a concentration dependent manner. Detection of g-tubulin was used to normalize quantifications. (C), EMSA on nuclear extracts from INS-1 cells transfected with 0, -1, -2- or 4 mg of plasmids encoding ICA512 (659-979)-GFP using as probe a biotynylated olinucleotide corresponding to GLE. The specific binding of the probe was verified by competition (comp.) with 15-fold molar excess of a non-biotynilated GLE oligo. (D), quantification of the EMSA result shown in (C) above. The results shown are representative of two independent experiments.

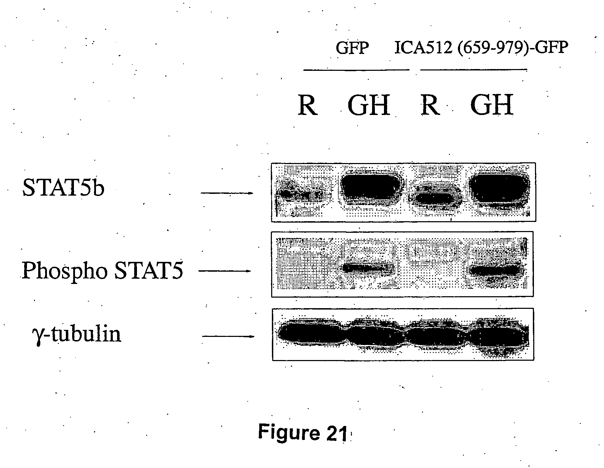

**Figure 21.** ICA512 increases Growth hormone induced phosphorylation of STAT5b.
   INS-1 cells were transfected with GFP or ICA512 (659-979)-GFP and grown for 48 h in complete media (RPMI containing 10% serum). Next, the cells were left 12 hrs in media without serum and then incubated for 1 h in resting buffer at 37 oC (5 mM KCl, 120 mM NaCl, 24 mM NaHCO3, 1 mM MgCl2, 2 mM CaCl2, 1 mg/ml ovalbumin, 15 mM HEPES pH 7.4). The cells were then incubated for additional 15 min in resting buffer with or without 20 nM growth hormone (Genotropin). The nuclear fraction was separated by SDS-PAGE and analyzed by western blot with antibodies against STAT5b, phospho-STAT5, and γ-tubulin.

The examples illustrate the invention.

### Example 1: Materials and Methods

**Islet isolation and cell culture.** Pancreatic islets were isolated from female Wistar rats by collagenase digestion, purified by density gradient centrifugation, and cultured (400/60 mm culture dish) as described previously (26). INS-1 cells were grown as described (27).

**cDNA cloning and transfection of INS-1 cells.** The cDNAs encoding full length human ICA512 and ICA512 (659-979) were amplified by PCR and subcloned into pEGFP-N1 or pECFP-N1 (Clontech, Palo Alto, CA) as EcoRl-Agel or Agel-Bglll inserts in frame with GFP or CFP, respectively. ICA512 was also tagged at the C-terminus with 3-HA epitopes by subcloning its cDNA as a Kpnl-Xbal insert into the MoHa3 vector (a kind gift from Monika Suchanek). Plasmids encoding the cDNAs of rat dynamin 1 and 2 or the dominant negative dynamin 1 and 2 (K44A) alleles fused to GFP were a kind gift from Kai Simons. Murine PIASy cDNA (a kind gift from Rudolf Grosschedl) was inserted as EcoRl-Bglll insert in pEYFP-N2 (Clontech). Plasmids encoding Ism4-CFP and Ism4-YFP were a kind gift from Karla Neugebauer. 4 x 106 INS-1 cells were electroporated with an Amaxa nucleoprator (Amaxa Biosystems, Cologne, Germany) using the nucleofector kit V and the program T-20. After electroporation, cells were aliquoted in two 35mm wells and grown for 4 days before being harvested. INS-1 cells stably expressing ICA512-GFP (INS-1 ICA512-GFP cells) were selected with 600µg/ml neomycin and cloned by limiting dilution. One out of 8 independent clones expressing ICA512-GFP, as assessed by western blotting and confocal microscopy, was selected for FACS using GFP fluorescence and further characterized.

**Cell extracts and western blotting.** Wild type and transfected INS-1 cells and rat pancreatic islets (350-450) were pre-incubated for 60 minutes in resting buffer containing 0 mM glucose and 5mM KCl and then incubated for various times in fresh resting or stimulating buffer including 25 mM glucose and 55 mM KCl, as previously described (7). The protease inhibitors calpeptin, MG-132, L-685,458 (Calbiochem, La Jolla, CA) were added in the last 15 minutes of the pre-incubation, and then for the entire following period before cells were harvested. Next, the cells were washed with ice cold PBS, and extracted in lysis buffer (10 mM Tris-HCl at pH 8.0, 140 mM NaCl, 1% Triton X-100, 1mM EDTA, 1 mM PMSF, and 1% protease inhibitor cocktail (Sigma, St. Louis, MO) at 4 °C. Cytosolic and nuclear extracts were prepared using the NE-PER Nuclear and Cytoplasmic Extraction Reagents (Pierce, Rockford, IL) in accordance with the manufacturer's instructions. Protein concentration was measured using the Bio-Rad Protein Assay Reagent (Bio-Rad, Hercules, CA). Equivalent amount of protein was separated by 8-10% SDS-PAGE, except for nuclear extracts, in which case half of the protein were loaded compared to the corresponding cytosolic extracts. After protein transferring, nitrocellulose filters were incubated with the following antibodies: mouse monoclonal anti-ICA512 (6), anti-µ-calpain (Chemicon, Temecula, CA), anti-γ-tubulin and anti-HA (Sigma), anti-GFP (Clontech); rabbit polyclonals anti-Glut2 (Alpha Diagnostics International, St Antonio, TX), anti-CPE (Chemicon), anti-GFP (Molecular Probes, Eugene, OR) and anti-HA (kind gift of Christoph Thiele). Chemiluminescence was developed using the Supersignal West Pico Substrate (Pierce) as substrate and detected with a LAS 3000 Bioimaging System (Fuji, Tokyo, Japan). Quantitation of protein signals was performed with the Image Gauge v3.45 software (Fuji).

**Time-course stimulation of INS-1 cells.** INS-1 cells were incubated for 1hr. in resting buffer (2.8 mM glucose and 5mM KCI), then for 6 hrs. in resting or stimulating buffer, with or without 40µM calpeptin, and then again in resting buffer for up to 24 hrs. Total RNA for real-time PCR was collected at time 0, 2 and 6 hrs. during stimulation and at 2, 6, 12 and 24 hrs. during the following resting period.

**Sucrose density gradient fractionation.** Fractionation of INS-1 and INS-1 ICA512-GFP cells on continuous sucrose density gradients from 0.4 - 2 M sucrose was performed as described (28,29). Fractions were separated by SDS-PAGE and analyzed by immunoblotting as indicated above.

**GST-Pull Down Assays.** ICA512 (601-979) cDNA in pET28a (Invitrogen) (29) was in vitro transcribed and translated using the T7/coupled transcription-translation system (Promega, Madison, WI) according to manufacture's instructions. PIAS1-GST and PIASy-GST (a kind gift of Rudolf Grosschedl) were expressed in bacteria as previously described (21, 30). Next, [³⁵S]-methionine-labeled ICA512 (601-979) was incubated overnight at 4 oC in GST binding buffer (120 mM NaCl, 50 mM Tris pH8, 0.25% Nonidet P-40, 1 mM DTT, 1mM PMSF) with GST-fusion proteins or GST alone coupled to glutathione-sepharose beads (Amersham, Freiburg, Germany). Beads were washed five times with binding buffer, and then eluted with 10 mM reduced glutathione. The eluate was subjected to SDS-PAGE and analyzed by autoradiography.

**In-vitro µ-calpain cleavage.** 3 µg bacterially expressed His-ICA512 cytoplasmic domain (residues 601-979) (Ort et al., 2001) were incubated in 20 µl buffer (20mM Tris-HCl pH 7.5, 50µM CaCl2, 1mM L-cysteine) at room temperature with or without 150 ng µ-calpain (Calbiochem) and 30 µM calpeptin or MG-132 for 1, 5, 15 or 45 minutes. The reactions were stopped by addition of an equal volume of 2x SDS sample buffer. Following SDS-PAGE, proteins were stained with Coomassie Blue (Bio-Rad).

**lmmunocytochemistry.** INS-1 cells in resting or stimulated conditions were fixed with 2% paraformaldehyde and then permeabilized with 0.2% saponin or 0.3% Triton X-100. After the permeabilization cells were incubated with mouse monoclonal anti- µ-calpain and insulin (Sigma) antibodies or rabbit polyclonal anti-ICA512ecto antibody (1) for 1 h. After washing and incubation with goat-anti-mouse Alexa⁴⁸⁸- or Alexa⁵⁶⁸-conjugated IgG (Molecular Probes), nuclei were counterstained with DAPI (Sigma). Images were acquired with an inverted LSM Meta 405nm confocal microscope (Zeiss, Göttingen, Germany) in 0.5 µm sections.

**Electronmicroscopy.** Cells were fixed with 4% PFA in 60 mM PIPES, 25 mM HEPES, 10 mM EDTA, 2 mM MgCl2, pH 6.9 for 1 hr. at room temperature, then scraped and pelleted in PBS with 10% gelatin. Pellets were fixed and stored in PBS with 1% PFA. Ultrathin cryosections were prepared as described (32) and incubated with mouse anti-insulin (Sigma, 1:1000) and rabbit anti-GFP (Molecular Probes, 1:500) antibodies followed by goat-anti-mouse and goat-anti-rabbit IgGs conjugated to 6 nm gold and 12nm gold particles, respectively (Dianova, 1:30).

**FRET analysis.** INS-1 cells transiently co-transfected with ICA512-CFP, PIASy-YFP, Ism4-YFP and Ism4-CFP were incubated either in resting or stimulating buffer for 90 minutes. Following fixation in 2% paraformahaldehyde, cells were imaged with an inverted LSM Meta 405nm confocal microscope (Zeiss) using a Plan-Apochromat 100X 1.4 oil DIC objective. The pinhole diameter was set between 1.0 and 1.25 Airy-Unit. Series of stack images with an optical thickness of 0.57 µm in the Z dimension were collected at a 8-bit intensity resolution over 512x512 pixels at pixel dwell time of 6.4 µsec. 458 nm and 514 nm laser lines were used to excite CFP and YFP, respectively. CFP images were acquired with a 458 nm dichroic mirror and a bandpass filter of 475-495, while YFP fluorescence was collected with a 514 nm dichroic mirror and a bandpass filter of 530-600 nm. FRET was assessed by measuring the dequenching of CFP during the photobleaching of YFP performed with the 514 nm laser line at 70% power and for six cycles of 30 sec. each. Mean fluorescence of CFP and YFP were determined by tracing the nuclear perimeter of the cell using the "region of interest" software function. Fluorescence intensity of CFP after photobleaching was normalized to 100% at time 0. In each experiment, 6-9 fields for each slide were photobleached and recorded. Each experiment was independently performed two times.

**Transferrin uptake assay**. INS-1 cells transfected with dynamin-GFP constructs and grown for 3-4 days on cover slips were starved for 1 h in medium without FBS, followed by 15 minutes incubation in serum free medium with transferrin conjugated with Alexa568 (Molecular Probes) and 1mg/ml BSA on 37 °C. Cells were washed 4x in cold PBS and fixed as indicated above. Images were acquired with CoolSnap-HQ CCD camera (Roper Scientific, Tuscon, AZ) attached to an Olympus BX61 microscope (Olympus, Tokyo, Japan) and processed with Metamorph 4.65 software (Universal Imaging, Downington, PA).

**RNA interference.** INS-1 cells (6x105/35-mm well) were grown for 2 days before transfection. 21-mer silecing RNA (siRNA) oligonucleotides 1, 2 and 3 for rat µ-calpain were synthesized with the Silencer siRNA Construction Kit (Ambion, Austin, TX) using the following primers: sense primer 1, 5'- AAGAGAGCGCTCCAGAACTCGCCTGTCTC-3'; antisense primer 1, 5'- AACGAGTTCTGGAGCGCTCTCCCTGTCTC-3'; sense primer 2, 5'- AACCTGGATCTGGTCATCTAGCCTGTCTC-3'; antisense primer 2, 5'-AACTAGATGACCAGATCCAGGCCTGTCTC-3'; sense primer 3, 5'-AAGTTACTGACCTCTCGAAGGCCTGTCTC-3'; antisense primer 3, 5'-AACCTTCGAGAGGTCAGTAACCCTGTCTC-3'. Cells were transfected on day 2 and day 4, each time with 1µg of the siRNA oligos/well in Optimem medium with Lipofectamine (Invitrogen). On day 6 cells were harvested and processed for RT-PCR or immunoblotting.

**Firefly luciferase assays.** Two days before transfection of siRNA oligos for firefly luciferase (8), INS-1 cells were co-transfected by electroporation with pGL3-Basic and phRL vectors (Promega) for co-expression of firefly and renilla luciferase, respectively. Firefly luciferase expression and activity were measured 6 days after plating of the cells.

**RT-PCR.** Cytosolic RNA was isolated using RNeasy Mini Kit (Qiagen, Hilden, Germany) according to the manufacturer's protocol. 1 µg of the isolated RNA was used as a template for the simultaneous amplification of rat µ-calpain and β-actin cDNA fragments by RT-PCR using the Titan One Tube RT-PCR System (Roche Diagnostics, Mannheim, Germany). For amplification of µ-calpain the following primers were used: sense, 5'-AACGAGTTCTGGAGCGCTCTC-3'; antisense, 5'-CTTCAGAATGATCTGGTAGAGG-3'. Sense and antisense primers (0.4 µM) for both genes were added in the same reaction. The number of cycles (32) had been optimized to be within the logarithmic phase of the reaction. PCR products were separated on 2% agarose gels, visualized with ethidium bromide and semiquantitated with the GeneSnap software (Syngene, Cambridge, UK) following image acquisition with GeneGnome (Syngene).

**Insulin RIA**. Cells were extracted overnight in 3:1:0.06 volumes of ethanol, H2O and 37% HCl at -20°C. Following centrifugation, total insulin content in the cells and in the medium was measured with the Sensitive Rat Insulin RIA Kit (Linco Research, St. Charles, MO). The stimulation index (SI) was calculated as follow: secreted insulin:cell insulin content + secreted insulin from stimulated cells / secreted insulin:cell insulin content + secreted insulin from resting cells. The stimulation index from control cells was equaled to 100%.

**RT-PCR**. Cytosolic RNA was isolated using RNeasy Mini Kit (Qiagen, Hilden, Germany) according to the manufacturer's protocol. 1µg RNA was used for the simultaneous amplification of rat µ-calpain and β-actin cDNAs by RT-PCR using the Titan One Tube RT-PCR System (Roche Diagnostics, Mannheim, Germany) and 0.4 µM primers for each gene. Primers for µ-calpain were as follows: sense, 5'-AACGAGT TCTGGAGCGCTCTC-3'; antisense, 5'-CTTCAGAATGATCTGGTAGAGG-3'. The number of cycles had been optimized to be within the logarithmic phase of the reaction. PCR products were separated on 2% agarose gels, stained with ethidium bromide and semiquantitated with the GeneSnap software (Syngene, Cambridge, UK) following image acquisition with GeneGnome (Syngene).

**Real-time PCR.** Total RNA from INS-1 cells was isolated with Trizol (Invitrogen, Karlsruhe, Germany) according to the manufacturer's protocol. 1µg of total RNA was used for the reverse transcriptase reaction, with 2 µM specific antisense primers for β-actin and insulin, as described (31). mRNA expression was measured by quantitative real-time PCR using the Brilliant SYBR Green QPCR (Stratagene, La Jolla, CA) with an Mx4000 Multiplex Quantitative Real Time PCR System (Stratagene).

### Example 2: Induction of ICA512 expression and cleavage by protease

In a first set of experiments, it was confirmed that pro-ICA512 is synthesized in a glucose-regulated fashion (see Fig. 1A lane 2, 4, 5 and 7). As stated above, processing of its ectodomain along the secretory pathway by a furin-like protease converts pro-ICA512 into its mature transmembrane form of 65 kD (ICA512-TMF), which is enriched on SGs (1). Stimulation of rat insulinoma INS-1 cells with high glucose (25 mM) and high K⁺ (55 mM) induces pro-ICA512 biosynthesis and reduces ICA512-TMF levels (Ort et al., 2001; and Fig. 1A and B, lanes 1 and 2). This dual effect on ICA512 can be dissociated by stimulating INS-1 cells either with high glucose or high K⁺ (33). High glucose alone prompts pro-ICA512 biosynthesis, but only a modest release of insulin and decrease of ICA512-TMF. High K⁺, on the other hand, induces a strong insulin secretion and decrease of ICA512-TMF, but it does not enhance pro-ICA512 biosynthesis. The reduction of ICA512-TMF depends on the potency of a stimulus in inducing SG exocytosis. Stimulation with high glucose alone for 30, 60 and 120 min. elicits indeed a progressive reduction of ICA512-TMF in freshly isolated rat pancreatic islets (Fig. 1C and D), which, unlike INS-1 cells, retain a virtually intact secretory responsiveness to glucose. The prompt induction of pro-ICA512 biosynthesis, a "signature" of the glucose stimulation, was less apparent in islets (Fig. 1E, lanes 4 and 2) than in INS-1 cells because the former convert pro-ICA512 into ICA512-TMF much more rapidly than the latter (8). Stimulation of SG exocytosis prompts the translocation of ICA512 to the plasma membrane (1) and reduces the levels of ICA512-TMF because of the Ca2+-dependent cleavage of its cytoplasmic domain by µ-calpain (7, and Fig. 1A and B, lanes 1-2). This cleavage, in particular, was prevented in a dose-dependent manner by calpeptin (Fig. 1A and B, lanes 3-5 and not shown), which inhibits calpain proteases (9), but not by L-685,458 (Fig. 1A and B, lanes 6-7), which selectively inhibits γ-secretase (10). A calpeptin-sensitive decrease of ICA512-TMF was also observed upon stimulation of purified rat pancreatic islets (Fig. 1E). Both in INS-1 cells and in pancreatic islets, on the other hand, we could not detect any cytosolic fragment resulting from the cleavage of ICA512-TMF. This is because our monoclonal anti-ICA512 antibody reacts with a juxtamembrane cytosolic epitope located between residues 643 and 663 (7), upstream of the calpain cleavage site (see below, and in particular Fig. 5A and B). Consistent with previous findings (7) was also the observation that the rapid biosynthesis of pro-ICA512 strictly depended on glucose, but not on the extent of ICA512-TMF cleavage.

Cleavage by µ-calpain was inhibited by calpeptin and MG-132, another inhibitor of cysteine proteases (FIG. 8). To establish whether µ-calpain is indeed responsible for the stimulus-induced proteolysis of ICA512-TMF in vivo, its cytoplasmic mRNA (Fig. 1F) and protein expression (Fig. 1G and H and FIG. 9) in INS-1 cells was transiently knockdown using three distinct silencing RNA (siRNA) oligonucleotides, alone (Fig. 1G and H) or in combination (FIG. 9). Decrease of µ-calpain by 37% inhibited by ~1.6 fold the stimulus-dependent degradation of ICA512-TMF (Fig. 1G and H), whereas knockdown of transiently transfected firefly luciferase, as verified by western blotting and enzymatic assay (Fig. 1I and J), had virtually no effect on µ-calpain or ICA512-TMF expression. Moreover, ICA512-TMF proteolysis was increased upon stimulation of INS-1 cells overexpressing µ-calpain (Fig.s 1K, 1L and 10). These data conclusively demonstrate that µ-calpain cleaves ICA512-TMF cytoplasmic domain upon stimulation.

µ-Calpain is a cytosolic protein that is recruited to the plasma membrane and activated by Ca2+ and phospholipids, both of which are directly implicated in the regulated exocytosis of SGs (13, 14). In INS-1 cells µ-calpain was mostly restricted to the plasma membrane region (Fig. 2A, left panel), where SGs were also enriched, as visualized by staining for ICA512 (Fig. 2A, middle panel). Upon subcellular fraction on sucrose density gradients the largest pool of µ-calpain was recovered in fractions containing the plasma membrane marker Glut-2 (Fig. 2B and C), while only a minor pool was detected in fractions enriched with ICA512 and carboxypeptidase E (CPE), another SG marker. To directly address whether µ-calpain-mediated cleavage of ICA512-TMF occurs prior or subsequent to SG exocytosis, INS-1 cells were transiently transfected with the light chain of clostridium tetanus toxin, which blocks the last step in the fusion of SGs with the plasma membrane by cleaving synaptobrevin 2/VAMP (15,16). Upon stimulation, ICA512-TMF cleavage (Fig. 3A, lane 3) and insulin secretion (Fig. 3C, lane 2) were similarly inhibited by transfection of the active form of tetanus toxin, but not by the transfection of its mutant, proteolytically inactive form (Fig. 3A, lane 4 and Fig. 3C, lane 3), thereby indicating that exocytosis of SGs and ICA512-TMF translocation to the plasma membrane are necessary in order for the latter to be susceptible to cleavage.

To test whether µ-calpain-mediated cleavage follows the internalization of ICA512-TMF, INS-1 cells were transiently transfected with green fluorescent protein (GFP)-tagged dominant negative mutant of dynamin 1 (K44A) or dynamin 2 (K44A), which block clathrin-mediated endocytosis (17). Expression of dynamin 1 (K44A)-GFP and wild-type dynamin 1-GFP had no significant effect on ICA512-TMF levels in resting INS-1 cells (Fig. 3D). In stimulated INS-1 cells, however, the expression of dynamin 1-GFP correlated with increased levels of ICA512-TMF compared to non-transfected cells (Fig. 3E, lanes 2 vs. 1), whereas expression of dynamin 1 (K44A)-GFP lead to an increased proteolysis of ICA512-TMF (Fig. 3E and F, lane 3), which was inhibited by calpeptin in a dose-dependent fashion (Fig. 3E and F, lanes 4-5). Similar results were observed in stimulated INS-1 cells transfected with wild type or dominant negative mutant dynamin 2-GFP (Fig. 3H and I). Control experiments showed that Alexa568-labeled transferrin was internalized in INS-1 cells non-transfected or transfected with dynamin 1-GFP or dynamin 2-GFP (Fig. 3G and FIG. 11, bottom panels), but not in those expressing dynamin 1 (K44A)-GFP or dynamin 2 (K44A)-GFP (Fig. 3G and FIG. 11 , top panels). Taken together, these results demonstrated that following stimulation and SG exocytosis ICA512-TMF is cleaved at the plasma membrane, where µ-calpain is recruited and activated by the increased Ca2+ levels, whereas endocytosis of ICA512-TMF spares it from cleavage.

### Example 3: Targeting of the C-terminus of ICA512 to the nucleus

In a next set of experiments, the fate of the ICA512 cleaved cytoplasmic fragment (ICA512-CCF) generated by µ-calpain was investigated. Since our monoclonal anti-ICA512 antibody reacts with an intracellular epitope located upstream of the cleavage site (data not shown), the C-terminus of ICA512 was tagged with GFP (ICA512-GFP) and stably expressed in INS-1 cells (ICA512-GFP INS-1 cells). Immunoblots with anti-GFP or anti-ICA512 antibodies on extracts from ICA512-GFP INS-1 cells showed that expression of pro-ICA512-GFP and ICA512-TMF-GFP at the expected molecular weights of 135 kD and 90 kD, respectively (Fig. 4A and B), while confocal microscopy and immunoelectron microscopy suggested that the large majority of ICA512-GFP colocalized with insulin (Fig. 4C and 4D). Enrichment of ICA512-GFP on SGs was confirmed by subcellular fractionation on sucrose density gradients (Fig. 4E). Moreover, the levels of ICA512-TMF-GFP were increased upon knockdown of µ-calpain by RNA interference (Fig. 4F and FIG. 12). Thus, GFP-tagging interfered neither with the conversion of pro-ICA512 into ICA512-TMF nor with the SG targeting and µ-calpain-mediated cleavage of the latter.

Western blotting with the anti-GFP antibody on cytosolic extracts from ICA512-GFP INS-1 cells showed the stimulation induced, calpeptin-sensitive proteolysis of ICA512-TMF-GFP (Fig. 5A, top panel, lanes 1-3). Strikingly, a GFP-reacting protein whose size of 55 kD matched the one predicted for ICA512-CCF-GFP was enriched in the nuclear, but not in the cytosolic fractions of stimulated cells, also in a calpeptin-sensitive manner (Fig. 5A, top panel, lanes 4-6). The inability of the anti-ICA512 monoclonal antibody to recognize this 55 kD protein further suggested its identity with ICA512-CCF-GFP (Fig. 5A, bottom panel). Notably, ICA512-CCF-GFP was already present in the nuclei of cells kept at rest for a cumulative time of 165 minutes. A likely explanation is that this interval was insufficient to clear the nuclei from ICA512-CCF-GFP continuously generated in cells grown with 11 mM glucose, which sub-maximally stimulates exocytosis.

Optical sectioning by confocal microscopy revealed the accumulation of GFP-positive dots in the nuclei of INS-1 ICA512-GFP cells stimulated for 105 minutes compared to those kept in resting conditions for an equivalent period of time (Fig. 5B). Concomitantly, the colocalization of ICA512-GFP and insulin in the cytosol was decreased, most likely reflecting the diminished insulin content and the internalization of ICA512-TMF-GFP in stimulated cells. The nuclear accumulation of ICA512-CCF-GFP in stimulated INS-1 cells was proportional to the increased proteolysis of ICA512-TMF-GFP over time (Fig. 5C and D), and was already detectable after 20' min. stimulation (Fig. 5D). While ICA512-CCF does not contain a canonic nuclear import signal, its nuclear translocation was unlikely the consequence of its fusion to GFP, since nuclear extracts of stimulated cells contained less GFP than those of resting cells (Fig. 5C and FIG. 13). To confirm the nuclear translocation of ICA512-CCF irrespectively of GFP, ICA512 was tagged at its C-terminus with three HA epitopes (ICA512-HA3) and transiently transfected in INS-1 cells. Western blotting with an anti-HA antibody confirmed the correct expression and processing of ICA512-HA3 in transfected cells (FIG. 14), while confocal microscopy revealed an increased punctuate reactivity of the anti-HA antibody in the nuclei of these cells upon stimulation (Fig. 5E). Immunoblotting also confirmed the enrichment of ICA512-HA3 in the nuclei of stimulated cells (Fig. 15). Taken together, these data demonstrated that SG exocytosis and ICA512-TMF cleavage at the plasma membrane is coupled to the generation of ICA512-CCF, which is targeted to the nucleus.

### Example 4: Determination of the functional role of ICA512-CCF

Subsequently, the role of ICA512-CCF was addressed. A two hybrid-screen in yeast showed that the cytoplasmic domain of ICA512 binds PIASy, thereby raising the possibility that ICA512-CCF modulates gene expression in β-cells. PIAS proteins have been identified as protein inhibitors of activated STAT (18) and more recently shown to have E3-sumoylating activity towards numerous substrates, including transcription factors (19-21). The specific interaction of ICA512 cytoplasmic domain with PIAS proteins, including PIASy and PIAS1, was confirmed by pull-down assay in-vitro (Fig. 6A). Specifically, 6 X histidine-tagged, 35S-labeled human ICA512 cytoplasmic domain (residues 601-979) generated by in-vitro transcription and translation was recovered on glutathione-sepharose beads coupled with PIASy-GST or PIAS1-GST, but not with GST alone (Fig. 6A). Likewise, ³⁵S-labeled PIASy interacts specifically with GST-ICA512(601-979) and GST-p53 (Fig. 6B). In INS-1 cells, as in other cell types, PIASy was restricted to the nucleus. The interaction in vivo of ICA512-CCF and PIASy in the nuclear compartment was confirmed by FRET in INS-1 cells transiently co-transfected with ICA512-CFP and PIASy-YFP (Fig. 6C). Specifically, the recovery of ICA512-CFP signal after photobleaching was increased -5 fold after stimulation (Fig. 6D). In parallel experiments, no FRET was detected between ICA512-CCF-CFP and the nuclear protein Ism4-YFP (Fig. 6D and FIG. 16) or between Ism4-CFP and PIASy-YFP (Fig. 6D) in either resting or stimulating conditions, thus confirming that ICA512-CCF and PIASy interact specifically in the nucleus.

To test whether ICA512-CCF is a retrograde signal that couples regulated secretion and gene expression, possibly through PIASy INS-1 cells were transiently transfected with a construct encoding the ICA512 cytoplasmic fragment generated in vitro by µ-calpain [ICA512 (659-979)] (7, and Fig. 8) fused at its C-terminus with GFP [ICA512 (659-979)-GFP]. ICA512 (659-979), which includes the tyrosine phosphatase-like domain, is either identical or closely mimics in length ICA512-CCF generated in vivo by µ-calpain. Analysis by confocal microscopy (Fig. 7A) and western blotting (Fig. 7B) showed that a fraction of ICA512 (659-979)-GFP is present in the nucleus of transfected INS-1 cells, and that its levels where comparable between resting and stimulated cells (Fig. 7B, lanes 1-2). Insulin mRNA, as measured by quantitative real-time PCR, was increased by 32% and 93% in resting INS-1 cells transiently transfected with ICA512-GFP or ICA512 (659-979)-GFP, respectively, whereas expression of GFP alone had virtually no effect on insulin mRNA levels (Fig. 7C). The overexpression of ICA512-GFP, and thereby the increased levels of ICA512-CCF-GFP, could account for the increment of insulin mRNA already in resting ICA512-GFP cells. Stimulation further increased by 42% the levels of insulin mRNA in ICA512-GFP expressing cells, while in cells expressing ICA512 (659-979)-GFP such increment was limited to 60%. These results are consistent with the fact that stimulation augments the levels of ICA512-CCF-GFP, but not those of ICA512 (659-979)-GFP. Furthermore, there was a correlation between the increased expression of ICA512 (659-979)-GFP and the enhancement of insulin mRNA levels (Fig. 7D). Accordingly, knockdown of µ-calpain decreases insulin mRNA (Fig. 7E). Likewise, calpeptin has an adverse effect on insulin mRNA in stimulated cells (Fig. 7F). In these experiments cells were either kept at rest or stimulated for up to 6 hrs., with or without 40µM calpeptin, and then returned to resting medium for up to 24 hrs. Maximal levels of insulin mRNAs are present 2 hrs. after the 6 hrs. stimulation period. In calpeptin-treated cells, however, insulin mRNA is reduced by ~40% at this time point as well as at any later time point compared to cells stimulated in the absence of calpeptin. The magnitude of this decrease correlates well with the degree of calpeptin-mediated inhibition of ICA512-TMF cleavage (Fig. 1 B). Taken together, these data show that upon translocation in the nucleus ICA512-CCF binds PIASy and up-regulates insulin mRNA levels.

The above explanations and data have identified a novel retrograde pathway that directly links regulated exocytosis and gene expression through the cleavage of a transmembrane protein associated with neuropeptide and in particular with insulin SGs. Previous studies have suggested that insulin itself may act as an autocrine signal through the activation on insulin receptors on b-cells (22). Further analyses, however, indicated that insulin receptors control islet development and the secretion (23), but not the expression (23, 24) of insulin. Similar to ICA512, receptor proteins such as Notch and the Alzheimer's disease amyloid precursor protein are proteolytically cleaved at the plasma membrane and then targeted to the nucleus for regulation of gene expression (25). The case of ICA512, however, is unique as its cleavage is directly coupled in time and space to the fusion of SGs with the plasma membrane. Moreover, such cleavage occurs in the cytoplasmic tail rather than in the transmembrane domain and is mediated by µ-calpain rather than by g-secretase.

Whereas the applicant does not wish to be bound by any scientific theory, a mechanistic model is proposed whereby cleavage of ICA512 upon SG exocytosis and translocation of its cytoplasmic tail to the nucleus allow β-cells to quantitatively monitor how many SGs underwent exocytosis in response to stimulation and promptly adjust their expression of insulin and presumably other genes involved in regulated secretion. As ICA512 is widely expressed in neuroendocrine cells, the novel teachings of the invention that give rise to the embodiments recited herein above and take into account for purposes of explanation only (but do not further depend on) such a mechanism will be of general relevance for peptide hormone and neuropeptide secretion.

### Example 5: ICA512 acts as a regulator of STATs in β-cells

In a further set of experiments, the mechanism by which ICA512 regulates gene expression has been investigated. First, the domain of ICA512 responsible for the interaction with PIASy has been mapped. As shown in Fig. 17, the PTP domain is required for binding to PIASy. Furthermore, it has been demonstrated that ICA512 is sumoylated by PIASy (see Fig. 18) and that the expression of ICA512 is modulated by the SUMO pathway (Fig. 19). Finally, it has been found that over-expression of ICA512-CCF enhances the levels of STAT5b and STAT3 in the nucleus (Fig. 20) and the tyrosine phosphorylation of STAT5b in response to stimulation with growth hormone (Fig. 21). The data in Fig. 20 furthermore show that over-expression of ICA512-CCF enhances the binding of STAT5 to the gamma-activated sequence-like DNA elements (GLE).
In summary, these data prove that ICA512 acts as a factor that enhances STAT signalling in relationship to β-cell secretory activity, and thereby promotes insulin gene expression an β-cell proliferation.

**Table 1**

| **Name** | **Abbreviation** | **Access Number** | **Database** |
|---|---|---|---|
| ADM precursor | ADML | P35318 | www.expasy.org |
| Agouti switch protein precursor | AGSW | P42127 | www.expasy.org |
| Agouti-related protein precursor | AGSR | 000253 | www.expasy.org |
| Apelin precursor | APEL | Q9ULZ1 | www.expasy.org |
| Atrial natriuretic factors | ANF | AAA51730 | www.ncbi.nlm.nih.gov/entre z |
| Beta-neoendorphin-dynorphin precursor | NDDB | P01213 | www.expasy.org |
| Brain natriuretic peptide precursor | ANFB | P13204 | www.expasy.org |
| Calcitonin gene-related peptide I precursor | CAL1 | Q99JA0 | www.expasy.org |
| Calcitonin gene-related peptide II precursor | CAL2 | P10092 | www.expasy.org |
| Calcitonin precursor | CAL0 | P01258 | www.expasy.org |
| Cholecystokinin precursor | CCKN | P06307 | www.expasy.org |
| Chromogranin A precursor | CMGA | P10645 | www.expasy.org |
| Cocaine- and amphetamine- | CART | Q16568 | www.expasy.org |
| regulated transcript protein precursor | | | |
| Corticoliberin precursor | CRF | P06850 | www.expasy.org |
| Corticotropin-lipotropin precursor | COLI | P01189 | www.expasy.org |
| Cortistatin precursor | CORT | 000230 | www.expasy.org |
| FMRFamide-related peptides precursor | NPFF | 015130 | www.expasy.org |
| FMRFamide-related peptides precursor | RFRP | Q9HCQ7 | www.expasy.org |
| Follistatin precursor | FSA | P19883 | www.expasy.org |
| Follitropin beta chain precursor | FSHB | P01225 | www.expasy.org |
| Galanin precursor | GALA | P22466 | www.expasy.org |
| Galanin-like peptide precursor | GALP | Q9UBC7 | www.expasy.org |
| Gastric inhibitory polypeptide precursor | GIP | P09681 | www.expasy.org |
| Gastrin precursor | GAST | P01350 | www.expasy.org |
| Gastrin-releasing peptide precursor | GRP | P07492 | www.expasy.org |
| Ghrelin precursor | GHRL | Q9UBU3 | www.expasy.org |
| Glucagon precursor | GLUC | P01275 | www.expasy.org |
| Glycoprotein hormones alpha chain precursor | GLHA | P01215 | www.expasy.org |
| Growth hormone variant precursor | SOM2 | P01242 | www.expasy.org |
| Insulin precursor | INS | P01308 | www.expasy.org |
| Insulin-like growth factor binding protein 3 precursor | IBP3 | P17936 | www.expasy.org |
| Insulin-like peptide INSL6 precursor | INL6 | Q9Y581 | www.expasy.org |
| Islet amyloid polypeptide precursor | IAPP | P10997 | www.expasy.org |
| Leydig insulin-like peptide precursor | INL3 | P51460 | www.expasy.org |
| Morphogenetic neuropeptide | MORN | P01163 | www.expasy.org |
| Motilin precursor | MOTI | P12872 | www.expasy.org |
| Neurexophilin 2 precursor | NXP2 | 095156 | www.expasy.org |
| Neurexophilin 3 precursor | NXP3 | 095157 | www.expasy.org |
| Neurexophilin 4 precursor | NXP4 | 095158 | www.expasy.org |
| Neuroendocrine protein 7B2 precursor | 7B2 | P05408 | www.expasy.org |
| Neurokinin B precursor | TKNK | Q9UHF0 | www.expasy.org |
| Neuromedin B-32 precursor | NEUB | P08949 | www.expasy.org |
| Neuromedin U-25 precursor | NEUU | P48645 | www.expasy.org |
| Neuropeptide B precursor | NPB | Q8NG41 | www.expasy.org |
| Neuropeptide W precursor | NPW | Q8N729 | www.expasy.org |
| Neuropeptide Y precursor | NEUY | P01303 | www.expasy.org |
| Neurotensin precursor | NEUT | P30990 | www.expasy.org |
| Nociceptin precursor | PNOC | Q13519 | www.expasy.org |
| Orexin precursor | OREX | O43612 | www.expasy.org |
| Oxytocin-neurophysin 1 precursor | NEU1 | P01178 | www.expasy.org |
| Pancreatic hormone precursor | PAHO | P01298 | www.expasy.org |
| Parathyroid hormone precursor | PTHY | P01270 | www.expasy.org |
| Parathyroid hormone-related protein precursor | PTHR | P12272 | www.expasy.org |
| Peptide YY precursor | PYY | P10082 | www.expasy.org |
| Pituitary adenylate cyclase activating polypeptide precursor | PACA | P18509 | www.expasy.org |
| Proenkephalin A precursor | PENK | P01210 | www.expasy.org |
| Progonadoliberin I precursor | GON1 | P01148 | www.expasy.org |
| Progonadoliberin II precursor | GON2 | O43555 | www.expasy.org |
| Prokineticin 2 precursor | PRK2 | Q9HC23 | www.expasy.org |
| Prolactin precursor | PRL | P01236 | www.expasy.org |
| Pro-MCH precursor | MLCH | P20382 | www.expasy.org |
| Prorelaxin H1 precursor | REL1 | P04808 | www.expasy.org |
| Protachykinin 1 precursor | TKN1 | P20366 | www.expasy.org |
| Protein-tyrosine phophatase-like N precursor | PTPN | Q16849 | www.expasy.org |
| Receptor-type protein-tyrosine phophatase N2 precursor | PTPX | Q92932 | www.expasy.org |
| Regulated endocrine specific protein 18 precursor | RP18 | P47940 | www.expasy.org |
| Resistin precursor | RSN | Q9HD89 | www.expasy.org |
| Resistin-like beta precursor | RSNB | Q9BQ08 | www.expasy.org |
| Secretin precursor | SECR | P09683 | www.expasy.org |
| Secretorygranin I precursor | SG1 | P05060 | www.expasy.org |
| Secretorygranin II precursor | SG2 | P13521 | www.expasy.org |
| Secretorygranin III precursor | SG3 | Q8WXD2 | www.expasy.org |
| Somatoliberin precursor | SLIB | P01286 | www.expasy.org |
| Somatostatin precursor | SMS | P01166 | www.expasy.org |
| Somatotropin precursor | SOMA | P01241 | www.expasy.org |
| Stanniocalcin 1 precursor | STC1 | P52823 | www.expasy.org |
| Stanniocalcin 2 precursor | STC2 | O76061 | www.expasy.org |
| Urocortin II precursor | UCN2 | Q96RP3 | www.expasy.org |
| Urocortin precursor | UCN1 | P55089 | www.expasy.org |
| Vasoactive intestinal peptide precursor | VIP | P01282 | www.expasy.org |
| Vasopressin-neurophysin 2-copeptin precursor | NEU2 | P01185 | www.expasy.org |

**Table 2**

| **Name** | **Associated disorders (proven or presumed)/use** |
|---|---|
| ADM precursor | pregnancy-induced hypertension, nonimmune hydrops fetalis |
| Agouti switch protein precursor | obesity, type II diabetes, increased tumor susceptibility, premature infertility |
| Agouti-related protein precursor | obesity, type II diabetes |
| Apelin precursor | anti-viral activity (?) |
| Atrial natriuretic factors | hypertension |
| Beta-neoendorphin-dynorphin precursor | temporal lobe epilepsy |
| Brain natriuretic peptide precursor | postmenopausal osteoporosis |
| Calcitonin gene-related peptide I precursor | osteoporosis, depression |
| Calcitonin gene-related peptide II precursor | osteoporosis |
| Calcitonin precursor | osteoporosis, depression |
| Cholecystokinin precursor | malabsorption syndrome, type II diabetes, Parkinson's disease |
| Chromogranin A precursor | unknown |
| Cocaine- and amphetamine-regulated transcript protein precursor | obesity, type II diabetes, anorexia |
| Corticoliberin precursor | depression. Alzheimer |
| Corticotropin-lipotropin precursor | obesity, type II diabetes. anorexia, Cushing syndrome, adrenal insufficiency |
| Cortistatin precursor | sleep disorders, depression |
| FMRFamide-related peptides precursor | pain and pressure control |
| FMRFamide-related peptides precursor | retinic macular dystrophy |
| Follistatin precursor | polycystic ovary syndrome |
| Follitropin beta chain precursor | amenhorrea, infertility |
| Galanin precursor | mammary gland trophisms |
| Galanin-like peptide precursor | unknown |
| Gastric inhibitory polypeptide precursor | type II diabetes |
| Gastrin precursor | Zollinger-Ellison syndrome |
| Gastrin-releasing peptide precursor | panic disorders |
| Ghrelin precursor | Prader-Willi syndrome, hyperphagia, obesity, type II diabetes, |
| Glucagon precursor | type II diabetes |
| Glycoprotein hormones alpha chain precursor | infertility, angiogenesis |
| Growth hormone variant precursor | growth hormone deficiency syndrome, dwarfism, acromegalia, |
| Insulin precursor | Type I and type II diabetes |
| Insulin-like growth factor binding protein 3 precursor | angiogenesis |
| Insulin-like peptide INSL6 precursor | unknown |
| Islet amyloid polypeptide precursor | type II diabetes |
| Leydig insulin-like peptide precursor | cryptorchidism, infertility, me |
| Morphogenetic neuropeptide | unknown |
| Motilin precursor | gastrointestinal motility disorders |
| Neurexophilin 2 precursor | unknown |
| Neurexophilin 3 precursor | unknown |
| Neurexophilin 4 precursor | unknown |
| Neuroendocrine protein 7B2 precursor | severe Cushing syndrome |
| Neurokinin B precursor | pain control, epilespy, diabetic retinopathy, angiogenesis |
| Neuromedin B-32 precursor | unknown |
| Neuromedin U-25 precursor | obesity, type II diabetes |
| Neuropeptide B precursor | pain control, obesity |
| Neuropeptide W precursor | unknown |
| Neuropeptide Y precursor | obesity, type II diabete, alcohol consumption, angiogenesis |
| Neurotensin precursor | maniac-depressive syndromes |
| Nociceptin precursor | pain control, inflammation, hearing impairment, memory |
| Orexin precursor | narcolepsy, sleep disorders, obesity |
| Oxytocin-neurophysin 1 precursor | social amnesia |
| Pancreatic hormone precursor | obesity, control of pancreatic secretion and biliary tract motility |
| Parathyroid hormone precursor | hypoparathyroidism, hypercalcemia |
| Parathyroid hormone-related protein precursor | hypercalcemia, psoriasis, bone and breast and tooth development |
| Peptide YY precursor | unknown |
| Pituitary adenylate cyclase activating polypeptide precursor | psychomotor disorders, glucohomeostasis |
| Proenkephalin A precursor | pain control, psychomotor disorders, anxiety |
| Progonadoliberin I precursor | hypogonadism, infertility, anosmia |
| Progonadoliberin II precursor | unknown |
| Prokineticin 2 precursor | control of behavioral circadian rhythm |
| Prolactin precursor | neurogenesis, lactation |
| Pro-MCH precursor | obesity, type II diabetes, |
| Prorelaxin H1 precursor | disorders of labor onset |
| Protachykinin 1 precursor | epilepsy |
| Protein-tyrosine phophatase-like N precursor | type II diabetes |
| Receptor-type protein-tyrosine phophatase N2 precursor | typell diabetes |
| Regulated endocrine specific protein 18 precursor | unknown |
| Resistin precursor | type II diabetes, obesity |
| Resistin-like beta precursor | allergic inflammation |
| Secretin precursor | promoting bile secretion |
| Secretorygranin I precursor | unknown |
| Secretorygranin II precursor | unknown |
| Secretorygranin III precursor | unknown |
| Somatoliberin precursor | dwarfism, achromegaly, cancer: prostate, digestive tract, others |
| Somatostatin precursor | neurogenesis |
| Somatotropin precursor | growth hormone deficiency syndrome, dwarfism, acromegalia, |
| Stanniocalcin 1 precursor | disorders of bone metabolism and kidney brain ischemia |
| Stanniocalcin 2 precursor | disorders of bone metabolism and kidney |
| Urocortin II precursor | hypertension, heart congestion |
| Urocortin precursor | obesity, stress, anxiety, hearing impairment |
| Vasoactive intestinal peptide precursor | diabetic retinopathy, rheaumatoid arthritis, inflammation |
| Vasopressin-neurophysin 2-copeptin precursor | diabetes insipidus |

### References

1. M. Solimena et al., EMBO J. **15**,2102 (1996).
2. D. U. Rabin et al., J. Immunol.**152**, 3183 (1994).
3. M. S. Lan, J. Lu, Y. Goto, A. L. Notkins, DNA Cell Biol. **13**, 505 (1994).
4. G. Magistrelli, S. Toma, A. Isacchi, Biochem Biophys Res Commun. **227**, 581 (1996).
5. K. Saeki et al., Diabetes **51**,1842 (2002).
6. J. M. Hermel, R. Dirkx Jr, M. Solimena, Eur. J. Neurosci. **11**, 2609 (1999).
7. T. Ort et al., EMBO J. **20**, 4013 (2001).
8. K. P. Knoch et al., Nat. Cell. Biol. **6,** 207 (2004).
9. T. Tsujinaka et al., Biochem. Biophys. Res. Commun. **153,** 1201 (1988).
10. M. S. Shearman et al., Biochemistry **39**, 8698 (2000).
11. A. Glading, D. A. Lauffenburger, A. Wells, Trends Cell Biol. **12**, 46 (2002).
12. P. Tompa et al., Nature **359**, 832 (1992).
13. T. M. F. Martin, Annu. Rev. Cell Dev. Biol. **14**, 231 (1998).
14. M. R. Wenk, P. De Camilli, Proc. Natl. Acad. Sci. U S A. (in press).
15. Schiavo G et al., Nature **359**, 832(1992).
16. R. Regazzi et al., EMBO J. **14**, 2723 (1995).
17. A. M. van der Bliek et al., J. Cell Biol. **122**, 553 (1993).
18. B. Liu et al., Proc. Natl. Acad. Sci. U S A. **95**, 10626 (1998).
19. E. S. Johnson, A. A. Gupta, Cell **106**, 735 (2001).
20. Y. Takahashi, T. Kahyo, A. Toh-E, H. Yasuda, Y. Kikuchi, J. Biol. Chem. **276,** 48973(2001).
21. D. Schmidt, S. Muller, Proc. Natl. Acad. Sci. U S A. **99**, 2872 (2002).
22. R. N. Kulkarni et al., Cell **96**, 329 (1999).
23. K. Otani et al., Am. J. Physiol. Endocrinol. Metab. **286**, E41 (2004).
24. B. Wicksteed, C. Alarcon, I. Briaud, M. K. Lingohr, C. J. Rhodes, J. Biol. Chem. **278**, 42080 (2003).
25. M. E. Fortini, Nat Rev Mol Cell Biol. **3,** 673 (2002).
26. M. Gotoh, T. Maki, T. Kiyoizumi, S. Satomi, A.P. Monaco, Transplantation **40**, 437 (1985).
27. M. Asfari et al., Endocrinology **130**, 167 (1992).
28. M. lezzi et al., Mol. Endocrinol. **13**, 202 (1999).
29. T. Ort et al., Eur. J. Cell Biol. **79**, 621 (2000).
30. S. Sachdev et al., Genes Dev. **15**, 3088 (2001).
31. H. Steinbrenner, T. B. Nguyen, U. Wohlrab, W. A. Scherbaum, J. Seissler, Endocrinology **143**, 3839 (2002).
32. C. E. Moolenaar et al., J. Cell Sci. **110**, 557 (1997).
33. T. Ort et al., EMBO J. **20,** 4013 (2001).

## Claims

1. A method for stimulating expression of peptide hormones in peptide-hormone secreting endocrine cells or neurons comprising the step of promoting in said cells or neurons the presence or activity
(aa) of (i) ICA512; or
(ii) a derivative thereof having ICA512 function; or
(iii) a fragment of ICA512 that may be cleaved by µ-calpain giving rise to a C-terminal fragment of ICA512 wherein said C-terminal fragment has the capability of being targeted to the nucleus; or
(iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or
(v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or
(vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or
(vii) a pro-form of any one of (i) to (vi); and
(ab) optionally of µ-calpain or a fragment or derivative thereof having µ-calpain function; or
(b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

2. A method of promoting cell proliferation of peptide-hormone secreting endocrine cells or neurons comprising the step of promoting in said cells or neurons the presence or activity
(aa) of (i) ICA512; or
(ii) a derivative thereof having ICA512 function; or
(iii) a fragment of ICA512 that may be cleaved by µ-calpain giving rise to a C-terminal fragment of ICA512 wherein said C-terminal fragment has the capability of being targeted to the nucleus; or
(iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or
(v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or
(vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or
(vii) a pro-form of any one of (i) to (vi); and
(ab) optionally of µ-calpain or a fragment or derivative thereof having µ-calpain function; or
(b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

3. The method of claim 1 or 2 wherein said PIAS protein is PIASy, PIAS1, PIAS3, PIASxα or PIASxβ.

4. The method of any one of claims 1 to 3 wherein the promotion comprises the transient or stable expression from an exogenously introduced vector of said
(aa) (i) ICA512; or
(ii) derivative thereof having ICA512 function; or
(iii) fragment of ICA512 that may be cleaved by µ-calpain giving rise to a C-terminal fragment of ICA512 wherein said C-terminal fragment has the capability of being targeted to the nucleus; or
(iv) derivative of said fragment of (iii) that may be cleaved by µ-calpain giving rise to a derivative of said C-terminal fragment of ICA512 wherein said derivative of said C-terminal fragment has the capability of being targeted to the nucleus; or
(v) fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or
(vi) fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or
(vii) pro-form of any one of (i) to (vi); and
(ab) optionally µ-calpain or a fragment or derivative thereof having µ-calpain function; or
(b) C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

5. The method of any one of claims 1 to 3 wherein the promotion comprises introducing into said cells or neurons said
(aa) (i) ICA512; or
(ii) derivative thereof having ICA512 function; or
(iii) fragment of ICA512 that may be cleaved by µ-calpain giving rise to a C-terminal fragment of ICA512 wherein said C-terminal fragment has the capability of being targeted to the nucleus; or
(iv) derivative of said fragment of (iii) that may be cleaved by µ-calpain giving rise to a derivative of said C-terminal fragment of ICA512 wherein said derivative of said C-terminal fragment has the capability of being targeted to the nucleus; or
(v) fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or
(vi) fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or
(vii) pro-form of any one of (i) to (vi); and
(ab) optionally µ-calpain or a fragment or derivative thereof having µ-calpain function; or
(b) C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

6. The method of any one of claims 1 to 3 wherein the promotion comprises reducing degradation or enhancing stability in said cells or neurons of said
(aa) (i) ICA512; or
(ii) derivative thereof having ICA512 function; or
(iii) fragment of ICA512 that may be cleaved by µ-calpain giving rise to a C-terminal fragment of ICA512 wherein said C-terminal fragment has the capability of being targeted to the nucleus; or
(iv) derivative of said fragment of (iii) that may be cleaved by µ-calpain giving rise to a derivative of said C-terminal fragment of ICA512 wherein said derivative of said C-terminal fragment has the capability of being targeted to the nucleus; or
(v) fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or
(vi) fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or
(vii) pro-form of any one of (i) to (vi); and
(ab) optionally µ-calpain or a fragment or derivative thereof having µ-calpain function; or
(b) C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

7. The method of any one of claims 1 to 6 wherein said C-terminal fragment of ICA512 consists of amino acids 659 to 979 of mature ICA512.

8. The method of any one of claims 1 to 7 wherein said endocrine cells are pancreatic β-cells.

9. The method of claim 8 wherein said promotion is effected by glucose, GLP-1 or calcium.

10. The method of any one of claims 1 to 9 wherein the peptide hormone or neuropeptide contained in said secretory granules is insulin, amylin or a peptide hormone or neuropeptide derived from one of the following precursors: ADM precursor, Agouti switch protein precursor, Agouti-related protein precursor, Apelin precursor, Atrial natriuretic factors, Beta-neoendorphin-dynorphin precursor, Brain natriuretic peptide precursor, Calcitonin gene-related peptide I precursor, Calcitonin gene-related peptide, II precursor, Calcitonin precursor, Cholecystokinin precursor, Chromogranin A precursor, Cocaine- and amphetamine-regulated transcript protein precursor, Corticoliberin precursor, Corticotropin-lipotropin precursor, Cortistatin precursor, FMRFamide-related peptides precursor, FMRFamide-related peptides precursor, Follistatin precursor, Follitropin beta chain precursor, Galanin precursor, Galanin-like peptide precursor, Gastric inhibitory polypeptide precursor, Gastrin precursor, Gastrin-releasing peptide precursor, Ghrelin precursor, Glucagon precursor, Glycoprotein hormones alpha chain precursor, Growth hormone variant precursor, Insulin precursor, Insulin-like growth factor binding protein 3 precursor, Insulin-like peptide INSL6 precursor, Islet amyloid polypeptide precursor, Leydig insulin-like peptide precursor, Morphogenetic neuropeptide, Motilin precursor, Neurexophilin 2 precursor, Neurexophilin 3 precursor, Neurexophilin 4 precursor, Neuroendocrine protein 7B2 precursor, Neurokinin B precursor, Neuromedin B-32 precursor, Neuromedin U-25 precursor, Neuropeptide B precursor, Neuropeptide W precursor, Neuropeptide Y precursor, Neurotensin precursor, Nociceptin precursor, Orexin precursor, Oxytocin-neurophysin 1 precursor, Pancreatic hormone precursor, Parathyroid hormone precursor, Parathyroid hormone-related protein precursor, Peptide YY precursor, Pituitary adenylate cyclase activating polypeptide precursor, Proenkephalin A precursor, Progonadoliberin I precursor, Progonadoliberin II precursor, Prokineticin 2 precursor, Prolactin precursor, Pro-MCH precursor, Prorelaxin H1 1 precursor, Protachykinin 1 precursor, Protein-tyrosine phophatase-like N precursor, Receptor-type protein-tyrosine phophatase N2 precursor, Regulated endocrine specific protein 18 precursor, Resistin precursor, Resistin-like beta precursor, Secretin precursor, Secretorygranin I precursor, Secretorygranin II precursor, Secretorygranin III precursor, Somatoliberin precursor, Somatostatin precursor, Somatotropin precursor, Stanniocalcin 1 precursor, Stanniocalcin 2 precursor, Urocortin II precursor, Urocortin precursor, Vasoactive intestinal peptide precursor, Vasopressin-neurophysin 2-copeptin precursor.

11. A method of treating or preventing type-1 or type-2 diabetes comprising stimulating expression of insulin in pancreatic β-cells wherein said stimulation comprises the step of promoting the presence or activity in said β-cells of
(aa) (i) ICA512; or
(ii) a derivative thereof having ICA512 function; or
(iii) a fragment of ICA512 that may be cleaved by µ-calpain giving rise to a C-terminal fragment of ICA512 wherein said C-terminal fragment has the capability of being targeted to the nucleus; or
(iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or
(v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or
(vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or
(vii) a pro-form of any one of (i) to (vi); and
(ab) optionally µ-calpain or a fragment or derivative thereof having µ-calpain function; or
(b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons.

12. Use of
(aa) (i) ICA512; or
(ii) a derivative thereof having ICA512 function; or
(iii) a fragment of ICA512 that may be cleaved by µ-calpain giving rise to a C-terminal fragment of ICA512 wherein said C-terminal fragment has the capability of being targeted to the nucleus; or
(iv) a derivative of said fragment of (iii) that may be cleaved by µ-calpain giving rise to a derivative of said C-terminal fragment of ICA512 wherein said derivative of said C-terminal fragment has the capability of being targeted to the nucleus; or
(v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or
(vi) a fragment or derivative of ICA512 that may be cleaved by µ-calpain giving rise to a C-terminal fragment of ICA512 or derivative thereof wherein said C-terminal fragment or derivative thereof has the capability of enhancing the nuclear levels, or tyrosine phosphorylation, or DNA binding activity of STATs in said cells or neurons; or
(vii) a pro-form of any one of (i) to (vi); and
(ab) optionally of µ-calpain or a fragment or derivative thereof having µ-calpain function; or
(b) of a C-terminal fragment of ICA512 or a derivative thereof which has the capability of being targeted to the nucleus or which has the capability of interacting with a PIAS protein or of enhancing the nuclear levels, or tyrosine phosphorylation, or DNA binding activity of STATs in said cells or neurons for the preparation of a pharmaceutical composition for treating or preventing type-1 or type-2 diabetes.

13. The use of claim 12 wherein said PIAS protein is PIASy, PIAS1, PIAS3, PIASxα or PIASxβ.

14. A method of screening for an agent capable of stimulating expression of peptide hormones in peptide hormone-secreting endocrine cells or neurons comprising the step of
(a) assessing expression of said peptide hormones in a test cell expressing
(aaa) (i) ICA512; or
(ii) a derivative thereof having ICA512 function; or
(iii) a fragment of ICA512 that may be cleaved by µ-calpain giving rise to a C-terminal fragment of ICA512 wherein said C-terminal fragment has the capability of being targeted to the nucleus and has the capability of interacting with a PIAS protein in said cells or neurons or/and has the capability of enhancing the nuclear levels, or tyrosine phosphorylation, or DNA binding activity of STATs in said cells or neurons; or
(iv) a derivative of said fragment of (iii) that may be cleaved by µ-calpain giving rise to a derivative of said C-terminal fragment of ICA512 wherein said derivative of said C-terminal fragment has the capability of being targeted to the nucleus and has the capability of interacting with a PIAS protein in said cells or neurons or/and has the capability of enhancing the nuclear levels, or tyrosine phosphorylation, or DNA binding activity of STATs in said cells or neurons; or
(v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or
(vi) a pro-form of any one of (i) to (v); and
(aab) µ-calpain or a fragment or derivative thereof having µ-calpain function; or
(ab) a C-terminal fragment of ICA512 or a derivative thereof which has the capability of interacting with a PIAS protein or/and the capability of being targeted to the nucleus or of enhancing the nuclear levels, or tyrosine phosphorylation, or DNA binding activity of STATs in said cells or neurons; and
(ac) a PIAS protein or a fragment or derivative thereof having PIAS function
in the presence and optionally in the absence of a test compound, wherein a higher level of expression in the presence of the test compound is indicative of its capacity to function as a stimulator of expression.

15. A method of screening for an agent capable of promoting cell proliferation of peptide hormone-secreting endocrine cells or neurons comprising the step of
(a) assessing expression of said peptide hormones in a test cell expressing
(aaa) (i) ICA512; or
(ii) a derivative thereof having ICA512 function; or
(iii) a fragment of ICA512 that may be cleaved by µ-calpain giving rise to a C-terminal fragment of ICA512 wherein said C-terminal fragment has the capability of being targeted to the nucleus and has the capability of interacting with a PIAS protein in said cells or neurons or/and has the capability of enhancing the nuclear levels, or tyrosine phosphorylation, or DNA binding activity of STATs in said cells or neurons; or
(iv) a derivative of said fragment of (iii) that may be cleaved by µ-calpain giving rise to a derivative of said C-terminal fragment of ICA512 wherein said derivative of said C-terminal fragment has the capability of being targeted to the nucleus and has the capability of interacting with a PIAS protein in said cells or neurons or/and has the capability of enhancing the nuclear levels, or tyrosine phosphorylation, or DNA binding activity of STATs in said cells or neurons; or
(v) a fragment or derivative of ICA512 that may be cleaved by µ-calpain giving rise to a C-terminal fragment of ICA512 or derivative thereof wherein said C-terminal fragment or derivative thereof has the capability of interacting with a PIAS protein in said cells or neurons; or
(vi) a pro-form of any one of (i) to (v); and
(aab) µ-calpain or a fragment or derivative thereof having µ-calpain function; or
(ab) a C-terminal fragment of ICA512 or a derivative thereof which has the capability of interacting with a PIAS protein or/and the capability of being targeted to the nucleus or of enhancing the nuclear levels, or tyrosine phosphorylation, or DNA binding activity of STATs in said cells or neurons; and
(ac) a PIAS protein or a fragment or derivative thereof having PIAS function
in the presence and optionally in the absence of a test compound, wherein a higher level of expression in the presence of the test compound is indicative of its capacity to function as a stimulator of proliferation.

16. A method of screening for an agent capable of stimulating expression of peptide hormones in peptide hormone-secreting endocrine cells or neurons comprising the step of
(a) assessing expression of said peptide hormones in a test cell expressing
(aa) a PIAS protein or a fragment or derivative thereof having PIAS function; or/and
(ab) a C-terminal fragment of ICA512 preferably consisting of amino acids 659 to 979 of mature ICA512
in the presence and optionally in the absence of a test compound, wherein a higher level of expression in the presence of the test compound is indicative of its capacity to function as a stimulator of expression.

17. A method of screening for an agent capable of promoting cell proliferation of peptide hormone-secreting endocrine cells or neurons comprising the step of
(a) assessing expression of said peptide hormones in a test cell expressing
(aa) a PIAS protein or a fragment or derivative thereof having PIAS function; or/and
(ab) a C-terminal fragment of ICA512 preferably consisting of amino acids 659 to 979 of mature ICA512
in the presence and optionally in the absence of a test compound, wherein a higher level of expression in the presence of the test compound is indicative of its capacity to function as a stimulator of cell proliferation.

18. A method of screening for an agent capable of stimulating expression of peptide hormones in peptide hormone-secreting endocrine cells or neurons comprising the step of
(a) assessing expression of said peptide hormones under the control of an inducible promoter in a non-human transgenic animal expressing
(aa) a PIAS protein or a fragment or derivative thereof having PIAS function; or/and
(ab) a C-terminal fragment of ICA512 preferably consisting of amino acids 659 to 979 of mature ICA512
in the presence and optionally in the absence of a test compound, upon induction of said promoter, wherein a higher level of expression in the presence of the test compound is indicative of its capacity to function as a stimulator of expression.

19. A method of screening for an agent capable of promoting cell proliferation of peptide hormones in peptide hormone-secreting endocrine cells or neurons comprising the step of
(a) assessing expression of said peptide hormones under the control of an inducible promoter in a non-human transgenic animal expressing
(aa) a PIAS protein or a fragment or derivative thereof having PIAS function; or/and
(ab) a C-terminal fragment of ICA512 preferably consisting of amino acids 659 to 979 of mature ICA512
in the presence and optionally in the absence of a test compound, upon induction of said promoter, wherein a higher level of expression in the presence of the test compound is indicative of its capacity to function as a stimulator of proliferation.

20. The method of any one of claims 14 to 19 wherein said test cell is a hormone-secreting endocrine cell or a neuron.

21. The method of any one of claims 14 to 20 wherein said endocrine cells are pancreatic β-cells.

22. The method of claim 21 wherein the secretory-peptide contained in said secretory granules is insulin.

23. A method of screening for an agent capable of stimulating expression of insulin in pancreatic β-cells comprising the step of assessing expression of insulin in a test cell expressing
(a) a PIAS protein or a fragment or derivative thereof having PIAS function; or/and
(b) a C-terminal fragment of ICA512 preferably consisting of amino acids 659 to 979 of mature ICA512
in the presence and optionally in the absence of a test compound, wherein a higher level of expression in the presence of the test compound is indicative of its capacity to function as a stimulator of insulin expression.

24. A method of screening for an agent capable of promoting cell proliferation of pancreatic β-cells comprising the step of assessing expression of insulin in a test cell expressing
(a) a PIAS protein or a fragment or derivative thereof having PIAS function; or/and
(b) a C-terminal fragment of ICA512 preferably consisting of amino acids 659 to 979 of mature ICA512
in the presence and optionally in the absence of a test compound, wherein a higher level of expression in the presence of the test compound is indicative of its capacity to function as a stimulator of cell proliferation.

25. The method of any one of claims 14 to 24 wherein said compound is a member of a library of compounds.

26. The method of claim 25 wherein said library of compounds is a library of small molecules, peptides, antibodies or antibody derivatives.

27. The method or use of any of the preceding claims wherein said derivative is a mimetic, preferably a peptidomimetic, a peptide aptamer or an anticalin.

28. The method of any one of claims 14 to 27 further comprising the step of testing in an animal model the efficacy of a compound assessed as being capable of stimulating the expression of said peptide hormones or promoting cell proliferation of peptide hormone secreting endocrine cells or neurons.


SEQUENCE LISTING

<110>  Technische Universität Dresden, Medizinische Fakultät Carl Gustav Carus

<120>  ICA512 Couples Insulin Secretion

<130>  K 1865 EP

<160>  2

<170>  PatentIn version 3.1

<210>  1
<211>  979
<212>  PRT
<213>  Homo sapiens

<400>  1
   Met Arg Arg Pro Arg Arg Pro Gly Gly Leu Gly Gly Ser Gly Gly Leu
   1               5                   10                  15
   Arg Leu Leu Leu Cys Leu Leu Leu Leu Ser Ser Arg Pro Gly Gly Cys
               20                  25                  30
   Ser Ala Val Ser Ala His Gly Cys Leu Phe Asp Arg Arg Leu Cys Ser
           35                  40                  45
   His Leu Glu Val Cys Ile Gln Asp Gly Leu Phe Gly Gln Cys Gln Val
       50                  55                  60
   Gly Val Gly Gln Ala Arg Pro Leu Leu Gln Val Thr Ser Pro Val Leu
   65                  70                  75                  80
   Gln Arg Leu Gln Gly Val Leu Arg Gln Leu Met Ser Gln Gly Leu Ser
                   85                  90                  95
   Trp His Asp Asp Leu Thr Gln Tyr Val Ile Ser Gln Glu Met Glu Arg
               100                 105                 110
   Ile Pro Arg Leu Arg Pro Pro Glu Pro Arg Pro Arg Asp Arg Ser Gly
           115                 120                 125
   Leu Ala Pro Lys Arg Pro Gly Pro Ala Gly Glu Leu Leu Leu Gln Asp
       130                 135                 140
   Ile Pro Thr Gly Ser Ala Pro Ala Ala Gln His Arg Leu Pro Gln Pro
   145                 150                 155                 160
   Pro Val Gly Lys Gly Gly Ala Gly Ala Ser Ser Ser Leu Ser Pro Leu
                   165                 170                 175
   Gln Ala Glu Leu Leu Pro Pro Leu Leu Glu His Leu Leu Leu Pro Pro
               180                 185                 190
   Gln Pro Pro His Pro Ser Leu Ser Tyr Glu Pro Ala Leu Leu Gln Pro
           195                 200                 205
   Tyr Leu Phe His Gln Phe Gly Ser Arg Asp Gly Ser Arg Val Ser Glu
       210                 215                 220
   Gly Ser Pro Gly Met Val Ser Val Gly Pro Leu Pro Lys Ala Glu Ala
   225                 230                 235                 240
   Pro Ala Leu Phe Ser Arg Thr Ala Ser Lys Gly Ile Phe Gly Asp His
                   245                 250                 255
   Pro Gly His Ser Tyr Gly Asp Leu Pro Gly Pro Ser Pro Ala Gln Leu
               260                 265                 270
   Phe Gln Asp Ser Gly Leu Leu Tyr Leu Ala Gln Glu Leu Pro Ala Pro
           275                 280                 285
   Ser Arg Ala Arg Val Pro Arg Leu Pro Glu Gln Gly Ser Ser Ser Arg
       290                 295                 300
   Ala Glu Asp Ser Pro Glu Gly Tyr Glu Lys Glu Gly Leu Gly Asp Arg
   305                 310                 315                 320
   Gly Glu Lys Pro Ala Ser Pro Ala Val Gln Pro Asp Ala Ala Leu Gln
                   325                 330                 335
   Arg Leu Ala Ala Val Leu Ala Gly Tyr Gly Val Glu Leu Arg Gln Leu
               340                 345                 350
   Thr Pro Glu Gln Leu Ser Thr Leu Leu Thr Leu Leu Gln Leu Leu Pro
           355                 360                 365
   Lys Gly Ala Gly Arg Asn Pro Gly Gly Val Val Asn Val Gly Ala Asp
       370                 375                 380
   Ile Lys Lys Thr Met Glu Gly Pro Val Glu Gly Arg Asp Thr Ala Glu
   385                 390                 395                 400
   Leu Pro Ala Arg Thr Ser Pro Met Pro Gly His Pro Thr Ala Ser Pro
                   405                 410                 415
   Thr Ser Ser Glu Val Gln Gln Val Pro Ser Pro Val Ser Ser Glu Pro
               420                 425                 430
   Pro Lys Ala Ala Arg Pro Pro Val Thr Pro Val Leu Leu Glu Lys Lys
           435                 440                 445
   Ser Pro Leu Gly Gln Ser Gln Pro Thr Val Ala Gly Gln Pro Ser Ala
       450                 455                 460
   Arg Pro Ala Ala Glu Glu Tyr Gly Tyr Ile Val Thr Asp Gln Lys Pro
   465                 470                 475                 480
   Leu Ser Leu Ala Ala Gly Val Lys Leu Leu Glu Ile Leu Ala Glu His
                   485                 490                 495
   Val His Met Ser Ser Gly Ser Phe Ile Asn Ile Ser Val Val Gly Pro
               500                 505                 510
   Ala Leu Thr Phe Arg Ile Arg His Asn Glu Gln Asn Leu Ser Leu Ala
           515                 520                 525
   Asp Val Thr Gln Gln Ala Gly Leu Val Lys Ser Glu Leu Glu Ala Gln
       530                 535                 540
   Thr Gly Leu Gln Ile Leu Gln Thr Gly Val Gly Gln Arg Glu Glu Ala
   545                 550                 555                 560
   Ala Ala Val Leu Pro Gln Thr Ala His Ser Thr Ser Pro Met Arg Ser
                   565                 570                 575
   Val Leu Leu Thr Leu Val Ala Leu Ala Gly Val Ala Gly Leu Leu Val
               580                 585                 590
   Ala Leu Ala Val Ala Leu Cys Val Arg Gln His Ala Arg Gln Gln Asp
           595                 600                 605
   Lys Glu Arg Leu Ala Ala Leu Gly Pro Glu Gly Ala His Gly Asp Thr
       610                 615                 620
   Thr Phe Glu Tyr Gln Asp Leu Cys Arg Gln His Met Ala Thr Lys Ser
   625                 630                 635                 640
   Leu Phe Asn Arg Ala Glu Gly Pro Pro Glu Pro Ser Arg Val Ser Ser
                   645                 650                 655
   Val Ser Ser Gln Phe Ser Asp Ala Ala Gln Ala Ser Pro Ser Ser His
               660                 665                 670
   Ser Ser Thr Pro Ser Trp Cys Glu Glu Pro Ala Gln Ala Asn Met Asp
           675                 680                 685
   Ile Ser Thr Gly His Met Ile Leu Ala Tyr Met Glu Asp His Leu Arg
       690                 695                 700
   Asn Arg Asp Arg Leu Ala Lys Glu Trp Gln Ala Leu Cys Ala Tyr Gln
   705                 710                 715                 720
   Ala Glu Pro Asn Thr Cys Ala Thr Ala Gln Gly Glu Gly Asn Ile Lys
                   725                 730                 735
   Lys Asn Arg His Pro Asp Phe Leu Pro Tyr Asp His Ala Arg Ile Lys
               740                 745                 750
   Leu Lys Val Glu Ser Ser Pro Ser Arg Ser Asp Tyr Ile Asn Ala Ser
           755                 760                 765
   Pro Ile Ile Glu His Asp Pro Arg Met Pro Ala Tyr Ile Ala Thr Gln
       770                 775                 780
   Gly Pro Leu Ser His Thr Ile Ala Asp Phe Trp Gln Met Val Trp Glu
   785                 790                 795                 800
   Ser Gly Cys Thr Val Ile Val Met Leu Thr Pro Leu Val Glu Asp Gly
                   805                 810                 815
   Val Lys Gln Cys Asp Arg Tyr Trp Pro Asp Glu Gly Ala Ser Leu Tyr
               820                 825                 830
   His Val Tyr Glu Val Asn Leu Val Ser Glu His Ile Trp Cys Glu Asp
           835                 840                 845
   Phe Leu Val Arg Ser Phe Tyr Leu Lys Asn Val Gln Thr Gln Glu Thr
       850                 855                 860
   Arg Thr Leu Thr Gln Phe His Phe Leu Ser Trp Pro Ala Glu Gly Thr
   865                 870                 875                 880
   Pro Ala Ser Thr Arg Pro Leu Leu Asp Phe Arg Arg Lys Val Asn Lys
                   885                 890                 895
   Cys Tyr Arg Gly Arg Ser Cys Pro Ile Ile Val His Cys Ser Asp Gly
               900                 905                 910
   Ala Gly Arg Thr Gly Thr Tyr Ile Leu Ile Asp Met Val Leu Asn Arg
           915                 920                 925
   Met Ala Lys Gly Val Lys Glu Ile Asp Ile Ala Ala Thr Leu Glu His
       930                 935                 940
   Val Arg Asp Gln Arg Pro Gly Leu Val Arg Ser Lys Asp Gln Phe Glu
   945                 950                 955                 960
   Phe Ala Leu Thr Ala Val Ala Glu Glu Val Asn Ala Ile Leu Lys Ala
                   965                 970                 975
   Leu Pro Gln

<210>  2
<211>  3613
<212>  DNA
<213>  Homo sapiens

<400>  2
cagcccctct ggcaggctcc cgccagcgtc gctgcggctc cggcccggga gcgagcgccc      60
ggagctcgga aagatgcggc gcccgcggcg gcctgggggt ctcgggggat ccgggggtct     120
ccggctgctc ctctgcctcc tgctgctgag cagccgcccg gggggctgca gcgccgttag     180
tgcccacggc tgtctatttg accgcaggct ctgctctcac ctggaagtct gtattcagga     240
tggcttgttt gggcagtgcc aggtgggagt ggggcaggcc cggccccttt tgcaagtcac     300
ctccccagtt ctccaacgct tacaaggtgt gctccgacaa ctcatgtccc aaggattgtc     360
ctggcacgat gacctcaccc agtatgtgat ctctcaggag atggagcgca tccccaggct     420
tcgcccccca gagccccgtc caagggacag gtctggcttg gcacccaaga gacctggtcc     480
tgctggagag ctgcttttac aggacatccc cactggctcc gcccctgctg cccagcatcg     540
gcttccacaa ccaccagtgg gcaaaggtgg agctggggcc agctcctctc tgtcccctct     600
gcaggctgag ctgctcccgc ctctcttgga gcacctgctg ctgcccccac agcctcccca     660
cccttcactg agttacgaac ctgccttgct gcagccctac ctgttccacc agtttggctc     720
ccgtgatggc tccagggtct cagagggctc cccagggatg gtcagtgtcg gccccctgcc     780
caaggctgaa gcccctgccc tcttcagcag aactgcctcc aagggcatat ttggggacca     840
ccctggccac tcctacgggg accttccagg gccttcacct gcccagcttt ttcaagactc     900
tgggctgctc tatctggccc aggagttgcc agcacccagc agggccaggg tgccaaggct     960
gccagagcaa gggagcagca gccgggcaga ggactcccca gagggctatg agaaggaagg    1020
actaggggat cgtggagaga agcctgcttc cccagctgtg cagccagatg cggctctgca    1080
gaggctggcc gctgtgctgg cgggctatgg ggtagagctg cgtcagctga cccctgagca    1140
gctctccaca ctcctgaccc tgctgcagct actgcccaag ggtgcaggaa gaaatccggg    1200
aggggttgta aatgttggag ctgatatcaa gaaaacaatg gaggggccgg tggagggcag    1260
agacacagca gagcttccag cccgcacatc ccccatgcct ggacacccca ctgccagccc    1320
tacctccagt gaagtccagc aggtgccaag ccctgtctcc tctgagcctc ccaaagctgc    1380

cagaccccct gtgacacctg tcctgctaga gaagaaaagc ccactgggcc agagccagcc 1440
cacggtggca ggacagccct cagcccgccc agcagcagag gaatatggct acatcgtcac 1500
tgatcagaag cccctgagcc tggctgcagg agtgaagctg ctggagatcc tggctgagca 1560
tgtgcacatg tcctcaggca gcttcatcaa catcagtgtg gtgggaccag ccctcacctt 1620
ccgcatccgg cacaatgagc agaacctgtc tttggctgat gtgacccaac aagcagggct 1680
ggtgaagtct gaactggaag cacagacagg gctccaaatc ttgcagacag gagtgggaca 1740
gagggaggag gcagctgcag tccttcccca aactgcgcac agcacctcac ccatgcgctc 1800
agtgctgctc actctggtgg ccctggcagg tgtggctggg ctgctggtgg ctctggctgt 1860
ggctctgtgt gtgcggcagc atgcgcggca gcaagacaag gagcgcctgg cagccctggg 1920
gcctgagggg gcccatggtg acactacctt tgagtaccag gacctgtgcc gccagcacat 1980
ggccacgaag tccttgttca accgggcaga gggtccaccg gagccttcac gggtgagcag 2040
tgtgtcctcc cagttcagcg acgcagccca ggccagcccc agctcccaca gcagcacccc 2100
gtcctggtgc gaggagccgg cccaagccaa catggacatc tccacgggac acatgattct 2160
ggcatacatg gaggatcacc tgcggaaccg ggaccgcctt gccaaggagt ggcaggccct 2220
ctgtgcctac caagcagagc caaacacctg tgccaccgcg cagggggagg gcaacatcaa 2280
aaagaaccgg catcctgact tcctgcccta tgaccatgcc cgcataaaac tgaaggtgga 2340
gagcagccct tctcggagcg attacatcaa cgccagcccc attattgagc atgaccctcg 2400
gatgccagcc tacatagcca cgcagggccc gctgtcccat accatcgcag acttctggca 2460
gatggtgtgg gagagcggct gcaccgtcat cgtcatgctg accccgctgg tggaggatgg 2520
tgtcaagcag tgtgaccgct actggccaga tgagggtgcc tccctctacc acgtatatga 2580
ggtgaacctg gtgtcggagc acatctggtg cgaggacttt ctggtgcgga gcttctacct 2640
gaagaacgtg cagacccagg agacgcgcac gctcacgcag ttccacttcc tcagctggcc 2700
ggcagagggc acaccggcct ccacgcggcc cctgctggac ttccgcagga aggtgaacaa 2760
gtgctaccgg ggccgctcct gccccatcat cgtgcactgc agtgatggtg cggggaggac 2820
cggcacctac atcctcatcg acatggtcct gaaccgcatg gcaaaaggag tgaaggagat 2880
tgacatcgct gccaccctgg agcatgtccg tgaccagcgg cctggccttg tccgctctaa 2940
ggaccagttt gaatttgccc tgacagccgt ggcggaggaa gtgaatgcca tcctcaaggc 3000
cctgccccag tgagaccctg gggccccttg gcgggcagcc cagcctctgt ccctctttgc 3060
ctgtgtgagc atctctgtgt acccactcct cactgcccca ccagccacct cttgggcatg 3120
ctcagccctt cctagaagag tcaggaaggg aaagccagaa ggggcacgcc tgcccagcct 3180
cgcatgccag agcctggggc atcccagagc ccagggcatc ccatgggggt gctgcagcca 3240
ggaggagagg aaaggacatg ggtagcaatt ctacccagag ccttctcctg cctacattcc 3300
ctggcctggc tctcctgtag ctctcctggg gttctgggag ttccctgaac atctgtgtgt 3360
gtccccctat gctccagtat ggaagaatgg ggtggagggt cgccacaccc ggctccccct 3420

gcttctcagc cccgggcctg cctctgactc acacttgggc gctctgccct ccctggcctc 3480
acgcccagcc tggtcccacc accctcccac catgcgctgc tcaacctctc tccttctggc 3540
gcaagagaac atttctagaa aaaactactt ttgtaccagt gtgaataaag ttagtgtgtt 3600
gtctgtgcag ctg 3613